# EUROPEAN PATENT APPLICATION

(11) **EP 1 652 530 A1**
(43) Date of publication of application: **03.05.2006**
(21) Application number: 04724124.5
(22) Date of filing: 29.03.2004
(51) Int. Cl.: A61K 38/17, A61K 47/24, A61P 35/00, A61P 37/04, A61P 31/00, A61P 43/00, C12N 15/12, C07K 14/47

(54) **DRUG PROMOTING CERAMIDE TRANSPORT, BASE SEQUENCE FOR PRODUCING THE DRUG, METHOD OF MEASURING ACTIVITY OF PROMOTING CERAMIDE RELEASE AND METHOD OF MEASURING ACTIVITY OF PROMOTING INTERMEMBRANE CERAMIDE TRANSFER**

(30) Priority: 14.07.2003 JP 2003274232
(71) Applicant: Japan Science and Technology Agency, Kawaguchi-shi, Saitama 332-0012 (JP); JAPAN as represented by DIRECTOR GENERAL OF NATIONAL INSTITUTE OF INFECTIOUS DISEASES, Tokyo 162-0052 (JP)
(72) Inventor: HANADA, Kentaro, Kawasaki-shi, Kanagawa 2130015 (JP); NISHIJIMA, Masahiro, Kawasaki-shi, Kanagawa 2160006 (JP); KUMAGAI, Keigo, Adachi-ku, Tokyo 1020006 (JP)
(74) Representative: Herzog, Martin
(86) International application number: PCT/JP2004/004455
(87) International publication number: WO 2005/004898

(57) **Abstract**

A drug for promoting ceramide transport contains a protein having the amino acid sequence of SEQ ID NO:1 as an effective component; the base sequence of SEQ ID NO:3; a method of measuring an activity for promoting ceramide release comprises mixing a lipidmembrane containing ceramide with a drugpromoting ceramide release, centrifuging the thus obtained mixture to give a supernatant and measuring the ceramide content in the supernatant; and a method of measuring an activity for promoting intermembrane ceramide transfer comprises mixing a receiving membrane, a drug for promoting ceramide transport and a donating membrane containing ceramide, adding a selective membrane aggregating agent thereto, then separating the receiving membrane from the donating membrane and measuring ceramide contents in the receiving membrane and in the donating membrane. Thus, it is possible to provide a drug for promoting ceramide transport, a base sequence for producing the drug, relevant a method of measuring an activity for promoting ceramide release and a method of measuring an activity for promoting intermembrane ceramide transfer.

## Description

### BACKGROUND OF THE INVENTION

### 1. FIELD OF THE INVENTION

The present invention relates to a drug for promoting ceramide transport, base sequence for manufacturing the relevant drug, a method of measuring activity for promoting ceramide release, and a method of measuring activity for promoting intermembrane ceramide transfer.

### 2. DESCRIPTION OF RELATED ART

Sphingolipid is a lipid ubiquitously existing in a eukaryotic organism. Sphingolipid plays an important role not only in a variety of cell function such as cell proliferation/differenciation, inflammatory reaction and cell death, but also in pathogen infection to the host cell, entry of toxin and the like. Hence, the discovery and/or invention of a protein and a chemical substance which have an influence on its metabolism and localization.

Ceramide is a molecule which is biosynthesized as an intermediate of sphingolipid synthesis, and is also a molecule generated by degradation of a complex sphingolipid. Ceramide in vivo attracts a great deal of attention, particularly from the viewpoint of the role of controlling cell proliferation and cell death and the role conducting the maintenance of water retention characteristics by the skin tissue as well as the role as a complex lipid synthesizing intermediate. Ceramide is extremely high in hydrophobic property and completely insoluble in water, its intermembrane transfer speed is extremely slow in the case where it is solely itself. Then, conventionally, in the case where ceramide is given to a cell, as described in non-patent documents 1-3, a short chain ceramide whose hydrophilic property is endowed by making it short acyl chain is alternately utilized or a natural type ceramide which has been dissolved in ethanol dodecane mixture as described in non-patent document 4 has been provided. However, in the former method, there is a problem that the short-chain ceramide only incompletely imitates the function of the natural type ceramide, on the other hand, in the latter method, there is a problem that the toxicity of an organic solvent becomes a large problem, respectively. In order to solve these problems, a method of giving ceramide to a cell by utilizing such a hydrophilic molecule that selectively catalyzing the intermembrane transfer of the natural type ceramide is considered. However, suchahydrophilicmolecule has neither been found nor invented yet.

In a cell, ceramide synthesized in the endoplasmic reticulum is efficiently transferred to the Golgi apparatus and converted into sphingomyelin(SM). However, it has been not clarified what kind of specific molecule is involved in the intermembrane transfer of ceramide.

Moreover, recently, it has been indicated that in the process of a cell death of glioma cell which is derived by nitrogen monoxide, the inhibition of ceramide transfer within a cell occurs. Hence, a molecule in a living organism specifically involved in a ceramide transfer is expected as a novel drug for controlling the life and death of the cell, however, as described above, a molecule in a living organism involving in a ceramide transfer has been not clarified yet.
Non-patent document 1: van Blitterswijk, W. J., van der Luit, A. H., Veldman, R. J., Verheij, M. and Borst, (J. Biochem. J. 369, 199-211, 2003)
Non-patent document 2: Hannun, Y. A. and Luberto, C. (Trends Cell Biol. 10, 73-80, 2000)
Non-patent document 3: Mathias, S., Pena, L.A. and Kolesnick, R. N. (Biochem. J. 335, 465-480, 1998)
Non-patent document 4: Ji, L., Zhang, G., Uematsu, S., Akahori, Y. and Hirabayashi, Y. (FEBS Lett. 358, 211-214., 1995)

Therefore, an obj ect of the present invention is to provide a drug for promoting ceramide transport, base sequence for manufacturing the relevant drug, a method of measuring activity for promoting ceramide release, and a method of measuring activity for promoting intermembrane ceramide transfer.

### SUMMARY OF THE INVENTION

In the circumstances, the present inventors have diligently studied and considered, and as a result of it, an animal culture cell mutationvariant (hereinafter, also referred to as LY-A strain) in which SM content is lowered since ceramide transfer within a cell is impaired is isolated, it is clarified that from the analysis using this variant, cytoplasmic protein is involved in ceramide transfer within a cell as an essential factor, and the conditions in which a cell whose SM content has been lowered is made selectively killed have been found, it has been acknowledged that under such conditions, in a method of selecting a functionally recovered strain from LY-A strain, that is, a functionally recovered strain whose SM content has been recovered for recovering the ceramide transfer within a cell, in the case where a separate splicing type product (hereinafter, may be referred to as GPBP Δ 26 protein) of Goodpasture antigen-binding protein (hereinafter, may be referred to as GPBP protein) and a protein whose sequence is essentially homologous (hereinafter, may be referred to as CERT protein) are contained, the function recovery strain is obtained, it has been found that a drug containing the relevant CERT protein as an effective component for promoting ceramide transfer within a cell, and the present invention has been completed.

Specifically, the present invention (1) a drug for promoting ceramide transfer which contains hCERT protein having an amino acid sequence of SEQ ID NO:1, hCERT_{L} protein having an amino acid sequence of SEQ ID NO:2, cCERT protein having an amino acid sequence of SEQ ID NO:3, and cCERT_{L} protein having an amino acid sequence of SEQ ID NO:4, or recombinant proteins of these described above as an effective component. By employing such a constitution, the present invention (1) exerts the effect that a novel drug for promoting ceramide transfer can be provided.

Moreover, the present invention (2) provides a drug described in the foregoing invention (1) which is a drug used as an antitumor agent, anti-inflammatory agent, organoregenesis agent, anti-infective agent, or a distribution promoting agent used for cosmetics. By employing such a constitution, the present invention (2) exerts an effect that a novel antitumor agent, anti-inflammatory agent, organo-regenesis agent, anti-infective agent, or a distribution promoting agent used for cosmetics can be provided in addition to the effect exerted by the foregoing invention (1).

Moreover, the present invention (3) provides a drug described in the foregoing invention (1) which is used for detecting a drug for inhibiting ceramide transfer. By employing such a constitution, the present invention (3) exerts an effect that a method of developing a novel drug can be provided, in addition to the effect exerted by the foregoing invention (1) .

Moreover, the present invention (4) provides a drug for promoting ceramide transfer described in the foregoing invention (1) whose effective component is a recombinant protein consisting of 370 residue to 598 residue of an amino acid sequence of SEQ ID NO:1 or 3, or 397 residue to 624 residue. By employing such a constitution, the present invention (4) exerts an effect that the activity for promoting ceramide transfer is significantly enhanced, in addition to the effect exerted by the foregoing invention (1).

Moreover, the present invention (5) provides a base sequence of SEQ ID NO:5, 6, 7 or 8 used for producing a drug described in the foregoing invention (1) or its recombinant base sequence.

Moreover, the present invention (6) provides a base sequence described in the foregoing invention (5) characterized in that a recombinant base sequence consists of 1108 base pair to 1794 base pair of the base sequence of SEQ ID NO:5, 1189 base pair to 1872 base pair of the base sequence of SEQ ID NO:6, 1539 base pair to 2225 base pair of the base sequence of SEQ ID NO:7, or 1189 base pair to 1872 base pair of the base sequence of SEQ ID NO:8.

Moreover, the present invention (7) provides a method of measuring the activity for promoting ceramide release in which an incubation process for incubating the mixture obtained by mixing a lipid membrane containing ceramide and a drug for promoting ceramide release is performed, a separating process for obtaining the supernatant from the mixture after it has been incubated by separating using centrifugation is performed, and subsequently, a quantification process for quantifying ceramide contained in the obtained supernatant. By employing such a constitution, the present invention (7) exerts an effect that a novel method of measuring the activity for promoting ceramide release can be provided.

Moreover, the present invention (8) provides a method of measuring the activity for promoting ceramide release described in the foregoing invention (7) in which a lipid membrane containing the foregoing ceramide has been prepared by adding ceramide to the mixed lipid of phosphatidylcholine and phosphatidylethanolamine. By employing such a constitution, the present invention (8) exerts an effect that the measurement of the activity for promoting ceramide release can be precisely performed in addition to the effect exerted by the foregoing invention (7).

Moreover, the present invention (9) provides a method of measuring the activity for promoting ceramide release described in the foregoing invention (7) or (8) in which a lipid membrane containing the foregoing ceramide has been subjected to a supersonic treatment. By employing such a constitution, the present invention (9) exerts an effect that a measurement of the activity for promoting ceramide release can be precisely performed, in addition to the effect exerted by the foregoing invention (7) or (8).

Moreover, the present invention (10) provides a method of measuring the activity for promoting ceramide release described in the foregoing invention (8) in which ceramide added to the lipid membrane containing the foregoing ceramide is a creamide radioactively labeled.By employing such a constitution, the present invention (10) exerts an effect that the quantification of ceramide in the quantification process can be easily performed, in addition to the effect exerted by the foregoing invention (8).

Moreover, the present invention (11) provides a method of measuring the activity for promoting the intermembrane transfer of ceramide in which an incubating process for mixing a receiving membrane, a drug for promoting ceramide transfer, a donating membrane and incubating the obtained mixture is performed, a separating process for separating the receiving membrane and the donating membrane by being subjected to a centrifugation after a membrane aggregating agent has been selectively added to the mixture obtained in the incubatingn process is performed, and a quantification process for quantifying ceramide contained by the separated receiving membrane and the donating membrane, respectively. By employing such a constitution, the present invention (11) exerts an effect that a novel method of measuring the activity for promoting the intermembrane transfer of ceramide.

Moreover, the present invention (12) provides a method of measuring the activity for promoting the intermembrane transfer of ceramide described in the foregoing invention (11) in which the foregoing receiving membrane has been prepared by the mixed lipid between phosphatidylcholine and phosphatidylethanolamine. By employing such a constitution, the present invention (12) exerts an effect that the measurement of the activity for promoting the intermembrane transfer of ceramide can be precisely performed.

Moreover, the present invention (13) provides a method of measuring the activity for promoting the intermembrane transfer of ceramide described in the foregoing invention (11) or (12) in which a donating membrane containing the foregoing ceramide is prepared by the mixed lipid containing phosphatidylcholine, phosphatidylethanol, lactocylceramide and ceramide. By employing such a constitution, the present invention (13) exerts an effect that the measurement of the activity for promoting the intermembrane transfer of ceramide, in addition to the effect exerted by the foregoing invention (11) or (12).

Moreover, the present invention (14) provides a method of measuring the activity for promoting the intermembrane transfer of ceramide described in the foregoing invention (11) in which ceramide added to the donating membrane containing the foregoing ceramide is a ceramide radioactively labeled. By employing such a constitution, the present invention (14) exerts an effect that the quantification of ceramide in the quantification process can be easily performed, in addition to the effect exerted by the foregoing invention.

Moreover, the present invention (15) provides a method of measuring the activity for promoting the intermembrane transfer of ceramide described in any one item of the foregoing inventions (11) to (14) in which the foregoing selective membrane aggregating agent is a castor seed lectin. By employing such a constitution, the present invention (15) exerts an effect that the separation in the separation process can be easily and swiftly performed, in addition to the effect exerted by the foregoing inventions (11) to (14).

### BRIEF DESCRIPTION OF THE DRAWING

For a more complete understanding of the present invention and the advantages thereof, reference is nowmade to the following detailed description taken in conjunction with the accompanying drawings, in which:
Fig. 1 is a diagram showing a domain structure and domain deletion structure of CERT protein;
Fig. 2 is a graphical representation showing the results of measuring the susceptibility with respect to MCD and lysenin of LY-A2 cell into which hCERT has been introduced using a retrovirus vector;
Fig. 3(A) is a graphical representation showing the reaction with respect to MCD and lysenin of LY-A2/hCERT cell;
Fig. 3(B) is a graphical representation showing phospholipid content of CHO-K1 cell, LY-A2 cell, or LY-A2/hCERT cell;
Fig. 4(A) is a diagram showing the results of lipid metabolism labeling experiment with [¹⁴C] serine in CHO-K1 cell, LY-A2 cell, or LY-A2/hCERT cell;
Fig. 4(B) is a diagram showing the results of lipid metabolism labeling experiment with [¹⁴C] choline in CHO-K1 cell, LY-A2 cell or LY-A2/h CERT cell;
Fig. 5 is a graphical representation showing the SM synthetic enzyme activity in CHO-K1 cell, LY-A2 cell, or LY-A2/hCERT cell;
Fig. 6 is a photography showing the results of cell labeling using C₅-DMB-Cer in CHO-K1 cell, LY-A2 cell, or LY-A2/hCERT cell and fluorescent microscopy observations;
Fig. 7 is a photography showing the results of cell labeling using C₅-DMB-Cer which has been performed for finding the influence of energy inhibitor on transfer within a cell of C₅-DMB-Cer in CHO-K1 cell, LY-A2 cell, or LY-A2/hCERT cell and fluorescent microscopy observations;
Fig. 8 is a graphical representation showing the results of a lipid metabolism experiment using radioactive serine performed for examining the influence of (1R, 3R) HPA-12 on SM new synthesis in CHO-k1 cell, LY-A2 cell, or LY-A2/hCERT cell;
Fig. 9(A) is a graphical representation showing MCD susceptibility of CHO-K1 cell, LY-A cell, or LY-A/hCERT cell;
Fig. 9(B) is a diagram showing the results of a lipid metabolism experiment using [¹⁴C] serine and [¹⁴C] choline in CHO-K1 cell, LY-A2 cell, or LY-A/hCERT cell;
Fig. 9(C) is a photograph showing the results of cell labeling using C₅-DMB-Cer in CHO-K1 cell, LY-A2 cell, or LY/hCERT cell and fluorescent microscopy observations;
Fig. 10(A) is a graphical representation showing the MCD susceptibility of (LY-A2+FL-hCERT) cell, (LY-A2+FL-hCERT_{L}) cell, (LY-A2+empty vector) cell, CHO-K1 cell, and LY-A2 cell;
Fig. 10(B) is a diagram showing the Western blot analysis using anti-FLAG antibody of (LY-A2+FL-hCERT) cell, (LY-A2+FL-hCERT_{L}) cell, (LY-A2+empty vector) cell;
Fig. 11 is a diagram showing the results of Northern blot analysis of mRNA coding CERT protein in CHO-K1 cell and LY-A cell;
Fig. 12(A) is a graphical representation showing the MCD sensibility of CHO-K1 cell, LY-A2 cell, (LY-A2+cCERT (G67E) -FL) cell and (LY-A2+cCERT-FL) cell;
Fig. 12(B) is a diagram showing the results of Western blot analysis using an anti-FLAG antibody;
Fig. 13(A) is a photography showing the results of localization analysis by microscopy observation of GFP or GFP fusion CERT expressed in (CHO-K1+pEGFP) cell, (CHO-K1+pcCERT-GFP) cell, (CHO-K1+pcCERT (G67E)-GFP) cell and Golgi apparatus localization marker;
Fig. 13(B) is a diagram showing the results of Western blot analysis using an anti-GFP antibody in CHO-K1 cell, (CHO-K1+pEGFP) cell, (CHO-K1+pcCERT-GFP) cell, (CHO-K1+pcCERT(G67E)-GFP) cell;
Fig. 14(A) is a graphical representation showing the degree of dependence on hCERT protein amount of the activity for promoting ceramide release;
Fig. 14(B) is a graphical representation showing the time dependency of the activity for promoting ceramide release;
Fig. 15 is a graphical representation showing the measurement results of the activity for promoting the releasing of ceramide, diacylglycerol, cholesterol, phosphatidylcholine, sphingomyelin, sphingosine;
Fig. 16 is a graphical representation showing the measurement results of ceramide release activity by hCERT protein, hCERT_{L} protein, hCERT Δ PH protein, hCERT Δ MR protein, andhCERT Δ ST protein, and further, PHhCERT protein, MRhCERT protein and SThCERT protein;
Fig. 17(A) is a graphical representation showing the degree of dependency on hCERT protein amount of the activity for promoting the intermembrane transfer of ceramide;
Fig. 17(B) is a graphical representation showing the time dependency of the activity for promoting the intermembrane transfer of ceramide;
Fig. 17(C) is a graphical representation showing the temperature dependency of the activity for promoting the intermembrane transfer of ceramide; and
Fig. 18 is a graphical representation showing the measurement results of the activity for promoting the intermembrane transfer of ceramide by hCERT protein, hCERT_{L} protein, hCERTΔ PH protein, hCERTΔ MR protein, and hCERTΔ ST protein, and further, PHhCERT protein, MRhCERT protein and SThCERT protein.

### DESCRIPTION OF THE PREFERRED EMBODIMENT(S)

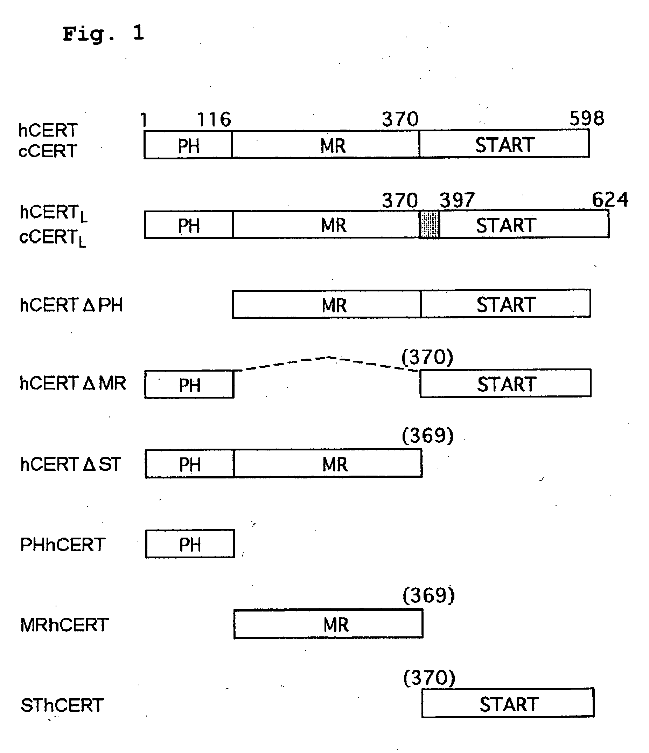

A drug of the present invention is composed of hCERT protein having an amino acid sequence of SEQ ID NO:1, hCERT_{L} protein having an amino acid sequence of SEQ ID NO: 2, cCERT protein having an amino acid sequence of SEQ ID NO:3, or cCERT_{L} protein having an amino acid sequence of SEQ ID NO:4, or the recombinant protein of these described above as an effective component. Domain deletion structures of hCERT protein, cCERT protein, hCERT_{L} protein and cCERT_{L} protein are shown in Fig. 1. As described in Fig. 1, it is considered that CERT protein has, if largely classified, three domains of pleckstrin homology(PH) domain at amino end about 120 amino acid region, steroidogenic acute regulatory protein-related lipid transfer(START) domain at carboxyl end about 230 amino acid region, and domain of these middle region (MR) . CERT protein and CERT_{L} protein described below maintain as high as 95% or more of amino acid sequence identity among mammal animals, respectively, and it is considered that a variety of natures that these proteins have are homologous in mammal animals in general. Hence, if concrete example has been presented by ones derived from human or CHO cell, it is not limited to these species. Herein, the term CERT protein is referred to a hCERT protein obtained from human, cCERT protein obtained from hamster cell, and an analogous protein obtained from the other mammal animal cell. The term CERT_{L} protein is referred to hCERT_{L} protein obtained from human cell, cCERT_{L} protein obtained from hamster cell and an analogous protein obtained from the cell of the other mammal animals.

Human CERT (hCERT) protein can be obtained by the following way. Complementary deoxyribonucleic acid(cDNA) that recovers SM content of LY-A strain is isolated and identified in accordance with the following procedure. After cDNA library derived from human cultured cell has been frequently and stably introduced into LY-A strain using retrovirus vector, the function recovery strain is isolated. The method of selecting a functionally recovered strain can be performed on the basis of the knowledge that the cell whose SM content has been decreased is highly sensitive to cholesterol drawing reagent/methylcyclodextrin(MCD), and the cell whose SM content has been recovered recovers the resistance against MCD. Subsequently, cDNA which has been introduced into the isolated functionally recovered strain was amplified by a genomic polymerase chain reaction(PCR) method, and retrieved. The retrieved cDNA changes the MCD sensitivity of LY-A strain into the level of a wild strain. The protein coded by cDNA described in SEQ ID NO:5 obtained in this way was the same with the protein published before as human GPBPΔ 26(hGPBPΔ 26). The function within the cell of GPBPΔ 26 has not been elucidated. As described in detail in the present specification, we have found that the function within the cell of GPBPΔ 26 is ceramide trafficking, therefore, as it is made nomenclature representing the function within a cell, the protein having substantially the same sequence with GPBPΔ 26 was named as CERT protein. Moreover, the protein having substantially the same with GPBP was named as CERT_{L} (a large splicing variant of CERT) . CERT protein can be obtained by expressing the isolated and identified cDNA, for example in bacterium such as E. coli, yeast and the like, insect cultured cell such as Sf9 cell and the like, mammal animal cultured cell such as CHO cell, HeLa cell, HEK293 cell and the like by a known method.

Human GPBP(hGPBP) has been reported as a protein for binding to carboxyl end sequence of α 3 chain of human collagen 4 type and phosphorylating it in cell-free system. Moreover, in the non-patent document 2, it has been reported that the separate splicing type product/hGPBPΔ 26 which is smaller than hGPBP by 26 amino acid also exists. From the analysis of transcription RNA level, it is indicated that both of hGPBP and hGPBPΔ 26 express in a variety of organs, however, it has been indicated that hGPBPΔ 26 dominantly expresses. However, hGPBP Δ 26 and hGPBP are localized mainly in cytoplasm, this localization is contradictory to the function expected first that collagen, that is an extracellular molecule is modified. Hence, it is considered that the physiological function of these proteins within a cell has not been elucidated from the studies achieved in the past, however, it is indicated that it is effective as a drug for promoting ceramide transport by the present inventors.

Moreover, as a recombinant protein of CERT protein, by making amino acid at N-end the first residue, START domain, that is, a fragment containing 371 residue-598 residue of SEQ ID NO:1 or 3, or 397 residue-624 residue of SEQ ID NO:2 or 4 can be listed, and as such a fragment, recombinant proteins obtained by expressing a DNA fragment obtained by an in vitro recombinant DNA method, a method of synthesizing, and in vivo recombination/gene recombination are listed. As such a protein, domain deletion protein such as PH domain deleted CERT Δ PH protein, MR domain deleted CERTΔ MR protein, ST CERT protein having only START domain and the like can be exemplified. It should be noted that it is estimated that lysine residue which is 370th amino acid of CERT protein corresponds to carboxyl end of MR domain. However, since there is a possible that the protein becomes unstable by the existence of lysine residue at the carboxyl end, in a domain deleted protein, the relevant lysine residue is incorporated at the amino end of START domain, and it is removed from the MR domain.

Furthermore, hGABPΔ 26 protein, that is, a recombinant protein whose amino acid base sequence is essentially the same with hCERT protein can be also obtained by expressing in accordance with a method of Raya, A et al(J. Biol. Chem. 274, 12642-12649, 1999) using plasmid vector obtained by a method of Raya, A et al(J. Biol. Chem. 275, 40392-40399, 2000) and purifying the generated product.

The sequence of cDNA of hGPBP, that is, a recombinant protein which is substantially the same with hCERT_{L} protein can be obtained by a known method (GenBank No: AF136450). Hence, DNA sequence of SEQ ID NO: 6 coding hCERT_{L} can be obtained by adding DNA sequence which is shorted to hCERT sequence by a PCR method. Then, hCERT_{L} protein can be obtained by expressing using plasmid vector prepared from the obtained cDNA and purifying the generated product. Moreover, the relevant hCERT_{L} protein can be also obtained by performing the expression using a plasmid vector prepared from cDNA in accordance with a method of Raya, A. et al(J. Biol. Chem. 274, 12642-12649, 1999).

Furthermore, the full length cDNA of CERT (cCERT) derived from CHO cell corresponding to base sequence of SEQ ID NO:7 can be determined by performing rapid amplification of cDNA ends(RACE) of cDNA end using SMART RACE cDNA amplifier kit manufactured by Clontech, Co., Ltd. Then, ORF of cCERT can be obtained by performing a PCR that CHO cell cDNA library has been made template. Moreover, DNA sequence for coding CERT_{L} (cCERT_{L}) derived from CHO cell corresponding to base sequence of SEQ ID NO:8 can be also amplified and cloned by this PCR. The amino acid sequence of cCERT_{L} is indicted in SEQ ID NO:4.

A drug of the present invention can be used as antitumor agent, anti-inflammatory agent, organoregenesis agent, or anti-infective agent by promoting or suppressing a cell death. Moreover, in cosmetics, as a distribution promoting agent of ceramide it can be used. Furthermore, it can be also utilized for inhibitor research of ceramide transport protein.

A drug of the present invention can be administered patenterally and orally by injection, rapid injection, nasopharynx absorption and percutaneous absorption. As a carrier preparation which is acceptable in a pharmaceutical preparation for parenteral administration, sterilized or aqueous or non-aqueous solution, suspended liquid and emulsion are listed. As an example of non-aqueous solvent, propylene glycol, pylyethylene glycol, a plant oil, for example, an olive oil, an injectable organic ester, for example ethyl oleate. A carrier for occluded bandage increases dermal permeability, then, it can be used for enhancing an antigen absorption. A liquid medication form for oral administration can be, in general, can include liposome solution containing a liquid medication form. As a suitable form for suspending liposome, inactive diluting agent generally used in the art, for example, an emulsion, a suspension, a solution a syrup and an elixir containing a purified water. Besides inactive diluting agent, such a composition can also contain an adjuvant, a wetting agent, an emulsified agent and a suspension stabilizer, and an edulcorant, a flavoring agent and perfume.

A drug of the present invention is also capable of containing an adjuvant. An adjuvant is a substance which can be used for non-specifically increasing the specific immune response. An adjuvant is classified into largely some groups.

As these groups, oil adjuvant (for example, Freund' s complete and incomplete adjuvant), inorganic salt (for example, AlK(SO4)₂), AlNH₄(SO₄)₂, AlNH₄(SO₄), silica, alum, Al(OH)₃, Ca₃(PO₄)₂, caolin and carbon), polynucleotide (for example, poly IC and poly AU acid), and a certain species of natural substance (forexample, wax D from Mycobacterium tuberculosis and substance found in members of Bordetella pertussis and Brucella family) are listed.

The present inventors have found that after a lipid membrane containing ceramide has been incubated with CERT protein, when it is centrifuged, ceramide remaining in a lipid membrane is sedimented, ceramide released from the lipid membrane transfers to the supernatant, and have invented a method of measuring the activity for promoting release of ceramide. Hereinafter, a method of measuring the activity for promoting ceramide release of the present invention will be explained. As a lipid membrane containing ceramide, it is not particularly limited, however, a lipid membrane in which ceramide has been added to the mixed lipid of phosphatidylcholine derived from egg yolk and phosphatidylethanolamine, or the mixed lipid of a synthetic phosphatidylcholine and a synthetic phosphatidylethanolamine and prepared can be exemplified. When a lipid membrane containing ceramide obtained in this way is sprayed by an inert gas such as nitrogen, argon or the like, or by drying under vacuum pressure, the exsiccation can be done. Moreover, it is also possible that a supersonic treatment can be performed by a method of performing the supersonic treatment by a bath type supersonic generator by adding a buffer such as Hepes-NaOH buffer in which NaCl and EDTA have been added to a lipid membrane or a lipid membrane dried under vacuum pressure, Tris-hydrochloric acid buffer or the like. As supersonic treatment conditions, for example, the conditions under which conditions of at 20 to 25°C for 3 to 6 minutes it is performed can be listed.

As ceramide that is added to the foregoing mixed lipid, a ceramide which has not been radioactively labeled may be available, however, if the convenience of measurement is considered, ceramide which has been radioactively labeled is preferable, as such a ceramide, ceramide which is radioactively labeled by ¹⁴C, ³H, ¹³N, ¹⁵O and the like can be listed, among these, ceramide radioactively labeled by ¹⁴C is preferable when its easy availability, stability and that the double labeling experiment with a separate lipid which has been ³H labeled can be performed.

As a drug for promoting ceramide release, it is not particularly limited, however, a drug for promoting ceramide transport of the above-described present invention can be exemplified.

In an incubating process, as a method of mixing a lipid membrane containing ceramide and a drug for promoting ceramide release, it is not particularly limited, however, a method that the foregoing lipid membrane is added to a buffer such as Hepes-NaOH buffer, Tris-hydrochloric acid buffer or the like into which the foregoing drug is dispersed can be listed. Moreover, as a method of incubating, a method of incubating at 15 to 42°C for 10 to 250 minutes by thermostatic bath, hot water bath or the like can be listed.

In a separate process, the supernatant is obtained by centrifuging the mixture after it has been incubated for 30 to 60 minutes at 50,000 xg.

In a quantifying process, in the case where ceramide is radioactively labeled, ceramide contained in the supernatant can be quantified by measuring the radioactivity of ceramide contained in the supernatant by means of liquid scintillation counter. Moreover, after a lipid contained in the supernatant has been separated by TLC,, it can be quantified by analyzing the radioactivity of ceramide using an image analyzer. In the case where ceramide has not been radioactively labeled, ceramide contained in the supernatant can be quantified by measuring by means of analysis using mass spectrometer or by means of a ceramide quantification method using E. coli diacylglycerol.

A method of measuring the activity for promoting the intermembrane transfer of ceramide of the present invention will be described below. As a donating membrane containing ceramide, it is not particularly limited, however, a donated membrane prepared by adding ceramide to the mixed lipid of phosphatidyl choline derived from egg yolk, phosphatidylethanolamine and lactocylceramide derived from pig tissue, or phosphatidylcholine, phosphatidylethanolamine and lactocylceramide which have been chemically synthesized can be exemplified. It is also possible that biotinated phosphatidylethanolamine is utilized instead of lactocylceramide. A donated membrane containing ceramide obtained in this way can be exsiccated by spraying an inert gas such as nitrogen, argon or the like or dried under vacuum pressure.

Moreover, it is also possible that a supersonic treatment can be performed by a method of performing the supersonic treatment by a bath-type supersonic generator by adding a buffer such as Hepes-NaOH buffer in which NaCl and EDTA have been added to a lipid membrane or a lipid membrane driedundervacuumpressure, Tris-hydrochloric acid buffer or the like. As supersonic treatment conditions, for example, the conditions under which conditions of at 20 to 25°C for 3 to 6 minutes it is performed can be listed.

As ceramide for adding to the foregoing mixed lipid, ceramide similar to a method of measuring the activity for promoting the above-described ceramide release can be listed.

As a receiving membrane, the mixed lipid of phosphatidylcholine derived from egg yolk, and phosphatidylethanolamine derived from egg yolk, or the mixed lipid of synthesized phosphatidylcholine and synthesized phosphatidylethanolamine or the like can be used.

As a method of mixing a drug for promoting ceramide transport and a donating membrane containing ceramide and a method of incubating the obtained mixture, it can be performed similar to the incubating process by a method of measuring the activity for promoting ceramide release.

As a selective membrane aggregating agent which is added to the mixture obtained in the incubating process, a castor seed lectin can be listed. Since a castor seed lectin is bound to galactocyl group contained in lactocyl ceramide, an aggregate consisting of a donating membrane and a castor seed lectin is formed, and only the donating membrane can be selectively precipitated by centrifuging at a low speed. In the case where biotinated phosphatidylethanolamine is utilized instead of lactocylceramide, biotin binding reagent such as avidin, streptoavidin and the like can be used as a selective membrane aggregating agent. Moreover, low-temperature insulation maintenance can be performed for 5 to 10 minutes at 0 to 4°C using ice bath, refrigerator or the like.

In a separate process for separating the mixture obtained in the cooling and maintaining process, the supernatant and precipitated donating membrane is obtained by centrifuging the mixture cooled after a selective membrane aggregating agent has been added for 3 to 10 minutes at 12,000xg. Moreover, the obtained donating membrane can be used by dissolving in SDS, octyl glucoside, chloroforme or the like in the quantifying process.

The quantifying process can be performed similar to a method of measuring the activity for promoting ceramide release.

### (EXAMPLES)

Hereinafter, the present invention will be further explained by exemplifying Examples, however, this is only exemplification, the present invention is not limited. It should be noted that in the following Examples, as far as the basic molecular biological operation is not particularly clearly indicated, it was performed by a method described in "Molecular Cloning", second edition, written by Sambrook, J., Fritsch, E. F. andmaniatis, T., published by Cold Spring Harbor Laboratory Press, in 1989, or "Current Protocol in Molecular Biology" written by Ausubel, F., Brent, R., Kingston, R., Moore, D., Seidman, J. , Smith, J. and Struhl, K., having been continuously published from 1987 up to the present by Wiley and Sons, or it was used in accordance with the instruction of the product which is commercially available in the market in the case where a reagent or a kit which is commercially available in the market.

### <Sample used in Examples>

- Human HeLa Retroviral library, pLIB vector, pLIB-EGFP, pEGFP-N3 and SMART™ RACE cDNA amplification kit, which have been manufactured by Clontech, Co., Ltd. were used.
- PcDNA3.1/Hyg(+), pcDNA3.1/Neo(+), LipofectAMINE PLUS™ reagent, pcDNA3.1/V5-His-TOPO(R) vector, pcR-Blunt II-TOPO (registered trade name) vector, SuperScript™ First Strand synthetic system, and synthetic oligonucleotide, which have been purchased from Invitrogen, Co., Ltd. were used,
- Nutridoma™ SP, FuGEGE™ 6 transfection reagent, Hygmycin B protease inhibitor cocktail (Complete™ EDTA-free) which have been purchased from Roche Applied Science, Co., Ltd. were used.
- LA PCR™ kit and Pyrobest (registered trade name) DNA polymerase which have been purchased from Takara, Co., Ltd. were used.
- KOD-Plus-DNA polymerase, which has been purchased from Toyobo, Co., Ltd. was used.
- pBlueScrip(R) SKII(pBS) vector, which has been purchased from Stratagene, Co., Ltd. was used.
- Wizard PLUS SV system which has been manufactured by Promega, Co., Ltd., it was used for purification in a small scale of plasmid.
- HiSpeed plasmid kit and genome chip system which have been manufactured by Qiagen, Co., Ltd. were used for purification in a large scale of plasmid and genome DNA purification, respectively.
- Restricted endonucleases which have been purchased f rom Takara, Co., Ltd., Toyobo, Co., Ltd., or New England Biolabs, Co., Ltd. were used.
- pET-28a (+) vector and E. coli B121 (DE3) strain which have been purchased for Novagen, Col., Ltd. were used.
- Alexa Fluor(registered trade name) 594 goat anti-mouse immuno-globulin G, 6-[N-(7-nitrobenzo-20oxa-1,3-diazol-4-yl)amino] caproyl-D-erythro-sphingosine; C₆-NBD-Cer and N-(4,4-difluoro-5, 7-dimethyl-4-bora-3a, 4a-diaza-s-indacene-3-pentanoyl)-(D)-erythro-sphingosine(N-(4,4-difluoro-5,7-dimethyl-4-bora-3a, 4a-diaza-s-indacene-3-pentanoyl)- D -erythro-sphingosine; C₅-DMB-Cer which have been purchased from Molecular Probe, Co., Ltd. were used.
- Methyl-beta-cyclodextrin(MCD), G418, polyprene, anti-FLAG (registered trade mark) M2 monoclonal antibody, and fatty acid already removed bovine serum albumin(BSA) which have been purchased from Sigma, Co., Ltd. were used
- Phosphatidyl choline derived from egg yolk, and phosphatidyl ethanol amine derived from egg yolk, which have been purchased from Avanti Polar Lipids, Co., Ltd. were used.
- Polyvinylidene difluoride(PVDF) membrane and horse radish peroxidase(HRP) binding goat anti-mouse immuno-globulin G, which have been purchased from Bio-Rad, Co., Ltd. were used.
- BD Living Colors™ A.v. monoclonal antibody, which has been purchased form Clontech, Co., Ltd. was used.
- N-palmitoyl-D-erythro-sphingosine(C16-ceramide) which has been purchased from BIOMOL Research Laboratories, Co., Ltd. was used.
- Lactocylceramide derived from pig tissue which has been purchased from Matreya, Co., Ltd. was used.
- A castor seed lectin(RCA120) which has been purchased from Honen, Co., Ltd. was used.
- A thin layer chromatography(TLC) plate which has been purchased from Merc, Co., Ltd. was used.
- 3-(4,5-dimethyl-thiazoyl)-2, 5-diphenyl-2H-tetrazolium bromide (MTT) which has been obtained from Dojin Chemical Institute, Co., Ltd. was used.
- Mouse anti-G28 monoclonal antibody which has been purchased from StressGen, Co., Ltd. was used.
- L-[2,3,4,5-³H]arginine(71Ci/mmol), L- [U-¹⁴C] serine (155 mCi/mmol), [Methyl-¹⁴C] Choline (55mCi/mmol), [1a,2a(n)-³H] cholesterol (49Ci/mmol), Megaprime DNA labeling kit and enhanced chemiluminescence (ECL) system which have been purchased from Amersham Bioscience, Co., Ltd. were used.
- [palmitoyl-1-¹⁴C] N-palmitoyl-D-erythro-sphingosin (55 mCi/mmol), [oleoil-1-¹⁴C] dioleoil-rac-glycerol (55 mCi/mmol), [choline methyl-¹⁴C]sphingomyelin (55 mCi/mmol), [dipalmitoyl-1-¹⁴C] L-α-dipalmitoyl phosphatidyl chloine (55 mCi/mmol), [2-palmitoyl-9, 10-³H (N)] L-α-dipalmitoyl phophatidyl choline (30 to 60 Ci/mmol), and D-erthythro- [3-³H] sphingosin (20 Ci/mmol) which have been purchased from American Radiolabeled Chemicals, Inc. were used. However, in the case where decomposed matter is mixed in [palmitoyl-1-¹⁴C] N-palmitoyl-D-erythro-sphingosin, after it has been purified by TLC, it was used.
- [α-³²P]dCTP (3000Ci/mmol) which has been purchased from PerkinElmer, Co., Ltd. was used.
- 5'-RACE Ready CHO cDNA and 3'-RACE Ready CHO cDNA prepared by utilizing SMART™ RACE cDNA amplification which have been allocated from Dr. Osamu Kuge of National Institute of Infectious Diseases, Department of Biochemistry and Cell Biology (at present, Kyushu University, Faculty and Graduate School of Sciences) were used.
- Lysenin which has been allocated from Dr. Yoshiyuki Sekizawa of Zenyaku Kogyo, Co., Ltd. was used.
- (1R,3R)N-(3-hydroxy-1-hydroxymethyl-3-phenyprol) dodecane amide [(1R, 3R) HPA-12] which has been allocated from Professor Shu Kobayashi of Graduate School of Pharmaceutical Sciences, The University of Tokyo was used.
- CHO cell cDNA library that has been prepared from mRNAderived from CHO-K1 cell using Superscript™plasmid system manufactured by Invitrogen, Co., Ltd. was used.
- PBS/nFL vector is a tailored vector derived from pBS, and PBS/nFL vector that the present inventors have prepared from pBS vector so that it has multi-cloning site to which FLAG™ epitope sequence can be added in amino end was used, however, the experimental operation performed using PBS/nFL vector described below in detail can be also performed using pBS vector.

### <Analyzing Device used in Examples>

- GeneAmp PCR system 2700 (registered trade name) and ABI PRIZM™ 310 which have been manufactured by Applied Biosystems, Co. , Ltd. were used for a PCR device and DNA sequence analyzing device.
- For image analysis of radioactive substance separated by TLC or the like, BAS2000 image analyzer of Fuji Film, Co., Ltd. was used.
- LSC-5100 manufactured by Aloka, Co., Ltd. was used for liquid scintillation counter, and Model F-3000 manufactured by Hitachi, Co., Ltd. was used for fluorescence spectrometer, respectively.
- Axiovert S100TV (registered trade name) of Carl Zeiss, Co., Ltd. was used for fluorescene microscopy, digital CCD camera C4742-95-12 manufactured by Hamamatsu Photonics, Co., Ltd. was used for charged coupled device (CCD).

### <PCR primer used in experiment>

PCR primers used in the present experiment are indicated in Table 1.

### Table 1

### <Method of culturing cell>

- CHO-K1 cell which has been purchased from American Type Cell Collection and has been subcultured and stored by the present inventors was used.
- CHO cell variant strain/LY-A strain that the present inventors, Hanada, K. , Hara, T. , Fukasawa, M. , Yamaj i, A. , Umeda, M. and Nishijima, M. (1998), J. Biol. Chem. 273, 33787-33794) have established were used. Plat-E cell which is a retrovirus packaging cell established by Kitamura et al (Gene Ther. 7, 1063-1066, 2000) and that has been allocated by Professor Toshio Kitamura of The Institute of Medical Science, The University of Tokyo who has established it was used.
- CHO cell was usually cultured in a cell culture medium (F12/NBS), in which Ham's F12 medium was supplemented with 10% newborn bovine serum (NBS), penicillin G (100 units/mL) and streptomycin sulfate (100 µg/mL), at the temperature of 33°C under 5% CO₂ atmosphere. In the case of Plat-E cell, it was cultured in a cell culture medium (DMEM/FBS), in which Dulbecco's minimum medium was supplemented with 10% fetal bovine serum (FBS), penicillin G (100units/mL) andstreptomycinsulfate (100µg/mL), at the temperature of 37°C under 5% CO₂ atmosphere.
- Moreover, Nutridoma medium (Ham's F12 medium containing 1% Nutridoma-SP and 25 µg/mL of gentamicin) or Nutridoma BO medium (medium inwhich 10 µM of sodium oleicacid/BAS complex and 0.1% FBS have been added in Nutridoma medium) which was prepared by Hanada et al (J. Biol. Chem. 267, 23527-23533., 1992) was used as a sphingolipid deficient medium.

### Examples of manufacturing

### <Manufacturing of protein which is an effective component of a drug of the present invention>

CERT protein and its recombinant protein were manufactured by the following procedure.

### <Method of establishing LY-A2 strain>

Since as for CHO cell, positive amino acid transporter (mCAT-1) which has been derived from mouse and is a mouse retrovirus receptor is not expressed, if it remains as it is, a mouse retrovirus vector cannot be infected. Then, the strain in which mCAT-1 cDNA has been stably expressed in LY-A strain (named as LY-A2 strain) was established as the followings. First, mCAT-1 cDNA which has been cut out by treating the restricted endonucleotide Stu I and Bam HI from plasmid pJET (donated from Dr. James Cunningham of Brigham & Women's Hospital in U.S.A.) described in Albritton, L. M. et al (Cell 57, 659-666, 1989) was inserted into Eco RV/Bam HI site of mammal animal cell expressing plasmid/pcDNA 3.1/Hyg(+) and mCAT-1/pcDNA 3.1/Hyg was prepared. After it has been treated with mCAT-1/pcDNA3.1/Hyg with Bg1 II and linearized, it was introduced into LY-A cell using lipofectAMINE PLUS reagent. After it has been cultured in F12/NBS medium in which hygromycin B (250 µg/mL) has been added and hygromycin B resistant cell has been selected, 6 pieces of hygromycin B resistant strain was purified by a limited-dilution method. The mCAT-1 expression level and its stability of these strains have been tested by making radioactive arginine uptake as an index, and the strain that expresses in the most stable state was made LY-A2 strain. The radioactive arginine uptake assay method carried out was in accordance with a method by Wang et al (J. Biol. Chem. 267, 23617-23624, 1992). However, as an uptake reaction liquid, a reaction liquid in which 115 mM KCl, 0.9 mM CaCl₂, 0.81 mM MgSO₄, 5 mM D-glucose and 0.1 mM L-[³H] arginine (1.25 µCi/mL) have been added was used, and the reaction was performed at 25°C for 30 seconds.

### <Method of preparing retrovirus particle for expressing human cDNA library>

A plasmid of a retrovirus library constructed from cDNA derived from HeLa cell was introduced into a Plat-E cell which is a packaging cell using a Fu' Gene transfection reagent. On the next day of the transfection treatment initiation, the medium was exchanged and further after it has been cultured for 24 hours, the medium was collected. The collected medium was, first, centrifuged at 150 xg for 5 minutes, and subsequently, the supernatant in which the supernatant has been centrifuged at 1350 xg for 5 minutes was made virus particle liquid. The prepared virus particle liquid has been cryopreserved at -80°C, and immediately before it was used, it was melted and used in an infection experiment.

### <Method of preparing retrovirus particle for expressing a cloned cDNA >

A plasmid in which the cDNA desired to express has been incorporated in a pLIB vector was introduced into a Plat-E cell by a method similar to the method described above. In order to express a green fluorescene protein (GFP), a virus paticle liquid was prepared by introducing pLIB-EGFP into Plat-E cell.

### <Method of infecting LY-A2 with retrovirus>

As a NBS used in a cell culture of infectious experiment, NBS which has been treated at 58°C for 30 minutes was used for the purpose of inactivating complement factor. 2 million pieces of LY-A2 cells were inoculated in a culture dish having the diameter of 100-mm and it has been cultured overnight at 37°C in 10 mL of F12/NBS. On the next day, human cDNA library expression retrovirus particle liquid was 1:10 diluted in F 12/NBS, and the liquid in which polybrene (800 µg/mL PBS) which has been filtered and sterilized was added so that the final concentration is 4 µg/mL was made infectious medium. The culture liquid of cell was exchanged with 5 mL per a culture dish of infectious medium and cultured at 37°C for 6 hours. The medium was exchanged again with 10 mL of F12/NBS, and further it has been cultured overnight. The cells were collected by a trypsin treatment 2 millions cells per a dish was inoculated in a 150-mm diameter culture dish. After it has been cultured at 33°C overnight in 10 mL of F12/NBS, it was subjected to MCD selection described in the next paragraph. It should be noted that from the preliminary experiment using a retrovirus particle liquid prepared from pLIB-EGFP vector, it was estimated that about 50% of LY-A2 cells are infected under the infectous conditions performed herein.

### <Method of selecting MCD resistance recovery strain>

The method of selecting function recovery strain used herein was performed on the basis of the knowledge that a cell whose SM content has been reduced becomes highly sensitive to cholesterol drawing reagent/methylcyclodextrin(MCD), and the cell whose SM content has been recovered recovers the resistance to MCD. As described in the foregoing paragraph, after the infected cell that has been seeded and cultured at 33°C overnight was washed twice in 10 mL of serum-free F12 medium, it has been at 37°C for one hour in 25 mL. After the culture dish was washed three times with 12 mL of PBS, 25 mL of F12/NBS was added and cultured at 33°C for 7 to 10 days. The surviving cell was inoculated by performing a trypsin treatment, and subjected to a similar MCD treatment. However, in the MCD treatments after the second treatment, the number of cells is small, therefore, the cell culture scale was made 60-mm diameter culture dish. Concerning with the still survived cell after the total 4 times of MCD treatments, the cell was purified by performing the limited-dilution. From the purified cells, the cell strain/LY-A2R cell whose MCD resistance, lysenin sensitivity and SM biosynthesis has been recovered to the level found in CHO-K1 cell was obtained.

### <Method of cloning by amplifying cDNA which has been introduced into LY-A2R cell>

The cDNA introduced into LY-A2R cell was amplified by genomic PCR,and cloned. Specifically, by making genomic DNA prepared from LY-A2R cell as a template, and by making a synthetic oligonucleotide corresponding to a sequence in a pLIB vector (primers #1 and #2 indicated in Table 2) as a primer, PCR was performed. DNA having about 2.4 kilo base pair (kbp) which has been amplified was inserted into pcDNA3.1/V5-His-TOPO vector. When the obtained plasmid was introduced into LY-A cell using LipofectAMINE PLUS reagent, the cell recovers to the CHO-K1 cell level was observed. When the sequence having the relevant 2.4 kbp DNA was determined, it was clarified that it is substantially the same with cDNA sequence (GenBank No: AF232930) of human GPBP Δ 26 protein which has been already reported by Raya, A et al (J. Biol. Chem. 275, 40392-40399, 2000). As already described in detail in the present specification, since it has been clarified that the function of this gene product within the cell is involved in ceramide transport, the present inventors have named the protein having essentially the same sequence with GPBP Δ 26 protein as CERT protein.

### <Method of cloning open-reading frame(ORF) of Human CERT>

ORF of human CERT protein was cloned by the following method. PCR was performed by making human HeLa retrovirus library manufactured by Clontech, Co., Ltd. as a template, and using the primers #3 and #5 indicated in Table 1. It should be noted that Eco RI site was added to the primer #3 which is a prospective primer and Xho I site has been added to the primer #5 which is a reversed primer, respectively. After DNA having about 1.8 kbp which has been amplified was treated with Eco RI and Xho I, the cloning was carried out by inserting it into Eco RI-Xho I site of pBS vector. The obtained plasmid was named as pBS/hCERT. It was admitted that the sequence of cDNA cloned herein is the same with ORF sequence of human GPBPΔ 26 protein (GenBank No:AF232930) already reported by Raya, Aetal (J. Biol. Chem. 275, 40392-40399, 2000).

### <Method of preparing DNA for coding human CERT_{L}>

It has been known that as a separate splicing type of GPBP Δ 26 protein (that is, substantially, CERT protein), exon having 78 bp has been further added and as a result of this, GPBP (that is, substantially, CERT_{L}) which is a product larger by 26 amino acid residue has expressed in a variety of species of cells. ORF sequence corresponding to human CERT_{L} (hCERT_{L}) was prepared as the followings, and the cloning was carried out. First, PCR was carried out by making pBS/hCERT as a template and using the primers #3 and #5 indicated in Table 1, and the amplified DNA having about 1.2 kbp was obtained. On the other hand, PCR was carried out by making pBS/hCERT as a template and using the primers #8 and #5 indicated in Table 1, and the amplified DNA having about 0.7 kbp was obtained. Next, PCR was carried out by making the protein in which these DNA having 1.2 kbp and DNA having 0.7 kbp have been mixed in the equimolar ratio as a template and using the primers #3 and #7 indicated in Table 1, and the amplified DNA having about 1.9 kbp was obtained. The plasmid cloned by inserting Eco RI and Xho I treatment products of this DNA having 1. 9 kbp into Eco RI-Xho I site of pBS vector was named as pBS/hCERT_{L}. It was admitted that the sequence of DNA having 1.9 kbp is corresponded with the ORF sequence of hGPBP protein (GenBank No: AF136450) which has been already reported by Raya, A et al (J. Biol. Chem. 274, 12642-12649, 1999).

### <Method of preparing DNA for coding a variety of deleted variants of CERT>

PCR was carried out by making pBS/hCERT as a template, and combining the primers indicated in Table 1 as the following, and the amplified DNA for coding a variety of deleted variants of CERT protein was obtained. For the purpose of obtaining the amplified DNA for coding PH domain deleted variant (hCERTΔ PH protein), and for the purpose of obtaining the amplified DNA for coding START domain deleted variant (hCERTΔ ST protein), the primers #3 and #10 are used, respectively. Moreover, for the purpose of obtaining the amplified DNA for coding MR domain deleted variant (hCERTΔ MR protein), after the product having about 350 bp by carrying out PCR using the primers #3 and #11 and the product having about 770 bp by carrying out using the primers #12 and #5 were obtained, respectively, PCR was carried out by making the one in which these PCR product DNAs have been mixed using the primers #3 and #5, the amplified DNA haovng about 1.1 kbp for coding hCERT Δ MR protein was obtained. The DNA for coding these deled variants was treated with Eco RI and Xho I and the cloning was carried out by inserting it into Eco RI-Xho I site of pBS vector. For the purpose of obtaining the amplified DNA for coding deleted variant having only PH domain (PHhCERT protein), for the purpose of obtaining the amplified DNA for coding deleted variant having only MR domain (MRhCERT protein), PCR product was obtained using the primers #15 and #16, for the purpose of obtaining the amplified DNA for coding deleted variant having only START domain (SThCERT protein), PCR product was obtained using the primers #17 and #18, respectively. The cloning was carried out by inserting Hind III and Xho I treatment product of these amplified DNA into Hind III-Xho I site of pBS/nFL vector.

### <Method of adding hCERT protein and hCERT_{L} protein of amino acid sequence which is capable of admitting by anti-FLAG antibody>

For the purpose of adding Asp-Tyr-Lys-Asp-Asp-Asp-Lys sequence (FL sequence) which is capable of admitting by anti-FLAG antibody at amino end of CERT, PCR was carried out by making pBS/hCERT as a template and using the primers #4 and #5 indicated in Table 1. The plasmid cloned by inserting Eco RI and Xho I treatment products of this amplified DNA into Eco RI-Xho I site of pBS vector was named as pBS/FL-hCERT. At the time when FL sequence is added to amino acid end of CERT_{L}, a similar PCR was carried out using pBS/hCERT_{L} as a template, and a plasmid in which the relevant product has been cloned was named as pBS/FL-hCERT_{L}.

### <Method of preparing plasmid which makes CERT protein and its related protein express in a mammal animal>

For the purpose of expressing via retrovirus vector infection, a variety of DNAs cloned at Eco RI-Xho I site of pBS vector was cut out by performing Eco RI-Xho I treatment, and the plasmid obtained by inserting these into Eco RI-Sal I site of pLIB vector for expressing retrovirus expression was used. This method can be also applied to a recombinant protein of CERT protein. On the other hand, for the purpose of expressing via lipofection, plasmid obtained by inserting DNA in which a variety of DNAs have been cloned into Eco RI-Xho I site of pBS vector was cut out by performing Eco RI-Xho I treatment into Eco RI-Xho I site of pcDNA3.1/Neo(+) vector was used. ORF of hCERT cloned by performing PCR amplication from HeLa cell cDNA library was inserted into pcDNA 3.1/Neo(+) vector and the plasmid pcDNA 3.1/hCERT was obtained.

### <Method of quantifying sensibility to MCD and lysenin>

100 thousand pieces of cells per each well of a 12-well culture dish were inoculated in 1 mL of F12/NBS culture medium and cultured at 33°C overnight. After Cells existed on the culture dish was washed twice in 1 mL of serum-free F12 medium, 1 mL of 10 mM MCD /F12 or lysenin (25 ng/mL) /F12 was added. It should be noted that culture medium in which 1 mL of only serum-free F12 has been added was made control. After it has been incubated at 37°C for one hour, it was washed twice in 1 mL of phosphate buffered saline (PBS). Subsequently, 1 mL of MTT (0.5 mg/mL)/F12 was added and incubated at 37°C for one hour. The relative amount of the generated reduced form of MTT was measured by a method of reference literature written by Hanada et al (J. Biol. Chem. 273, 33787-33794, 1998).

### <Method of introducing gene for coding CERT protein into CHO cell, and method of separating stabilized expression strain >

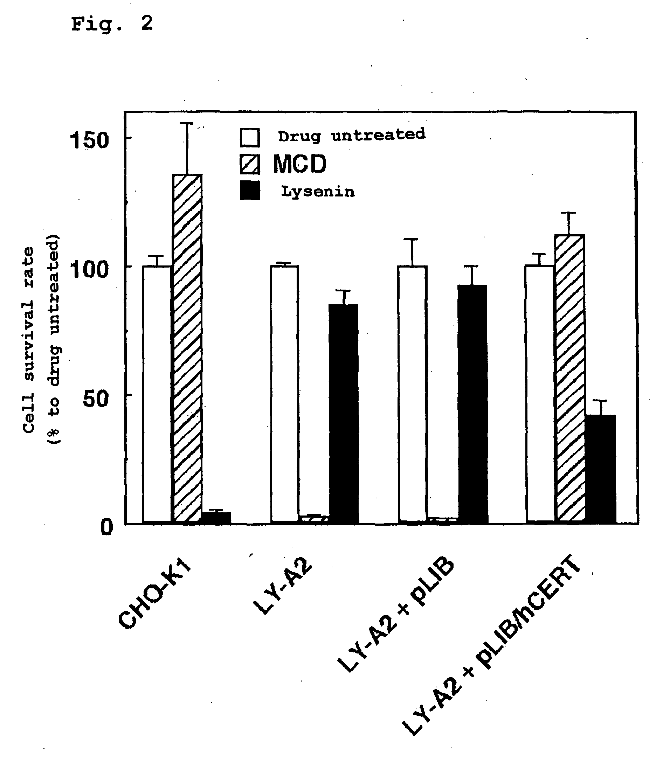

The relevant gene was expressed in LY-A2 cell by making virus particle derived from retrovirus vector infect it prepared in the foregoing paragraph. Specifically, the relevant gene was expressed in LY-A2 cell by making virus particle derived from pLIB/hCERT. In the case where hCERT was expressed, the purified transformed strain was also separated by a ultradilution method, and this strain was nambed as LY-A2/hCERT. After it has been cultured for days, the sensitivity to MCD and lysenin was examined by the above-described method. As a control, also concerning with the cell that virus particle derived from pLIB vector in which cDNA has not inserted infects LY-A2 cell, the sensibility of it to MCD and lysenin was examined. Furthermore, also concerning with CHO-K1 cell, the sensitivity of it to MCD and lysenin was examined. The results of the sensitivity measurement to MCD and lysenin are indicated in Fig. 2.

Moreover, the relevant gene was expressed by introducing the expressed plasmid derived from pcDNA 3.1/Neo(+) prepared in the foregoing paragraph in LY-A cell using LipofectAMINE PLUS reagent. Specifically, the relevant gene was expressed in LY-A cell by introducing pcDNA 3.1/hCERT. For the purpose of purifying the stably expressed strain of hCERT, first, G418 resistant cell was selected and it was purified by a ultradilution method. Then, the sensibility of it to MCD and lysenin was examined by the above-described method, and the strain whose MCD sensibility has been recovered to the wild type level was separated. This transformed strain was nambed as LY-A/hCERT.

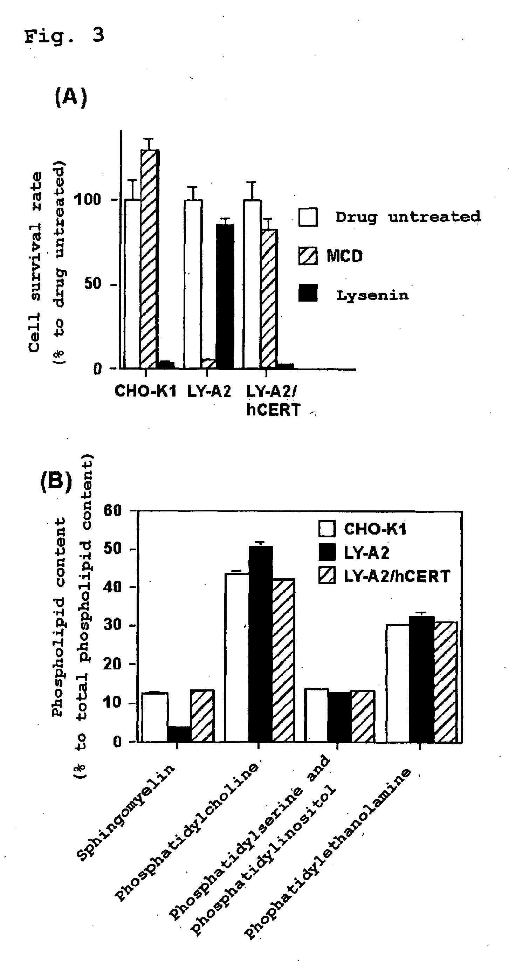

From LY-A2 cell infected with virus particle derived from pLIB/hCERT, transformed strain was purified and the reactivity of LY-A2/hCERT cell which is a purified strain with respect to MCD and lysenin was further examined in detail. The results are indicated in Fig. 3(A).

### <Method of determining content of a variety of phospholipids of cell>

3 x 10⁶ cells per culture dish having the 150-mm diameter of CHO-K1 cell, LY-A2 cell, or LY-A2/hCERT cell was inoculated in 20 mL of F12/NBS medium, and cultured at 33°C overnight. After it was washed twice in 10 mL of serum-free F12 medium, it was further cultured for 2 days in 20 mL of Nutridoma B0 medium. After it was washed in PBS, the cells were collected by a method of scaping, suspended again in PBS, and lipid was extracted by a method described in Bligh et al (Can. J. Biochem. Physiol. 37, 911-917, 1959) . The extracted lipid was separated using TLC whose development solvents were chloroform/methanol/acetic acid/H₂O (volume ratio, 25: 15: 4: 2). After the separated phospholipid on the TLC plate was colored by iodine vapor, the band of the respective separated phospholipid was scraped out from the plate. The scraped separated phospholipidwas quantified by measuring phosphorus content by a method of Rouser et al (Lip ids 1, 85-86, 1966). The results are indicated in Fig. 3 (B).

### <Metabolism labeling experiment method of lipid using [¹⁴C] serine and [¹⁴C] choline>

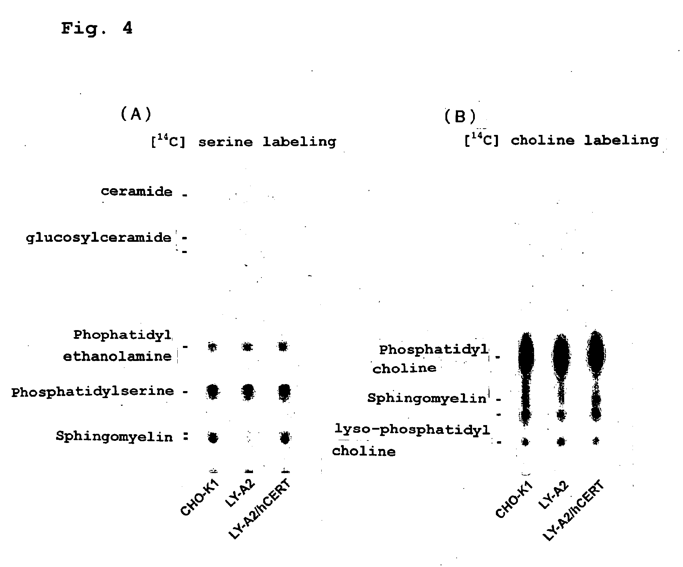

CHO-K1 cell, LY-A2 cell, and LY-A2/hCERT cell were inoculated at the cell density of 1.0 x 10⁶ per culture dish having the 60-mm diameter, and cultured at 33°C overnight. Subsequently, it was exchanged with 15 mL of Nutridoma medium, after [¹⁴C] serine (0.75 µCi) or [¹⁴C] choline (1.0 µCi) have been added, in the case where [¹⁴C] serine is added, it was cultured for 2 hours, and in the case where [¹⁴C] choline is added, it was cultured at 33°C for 5 hours. However, in the case where the influence on (1R, 3R) HPA-12 is analyzed, after it was pretreated at 4°C for 15 minutes in 1.5 mL of Nutridoma medium to which 1 µM of (1R,3R) HPA-12 has been added (in control experiment, dimethylsulfoxide used for dissolving a drug was added at the concentration corresponding to 0.01% of the final concentration), [¹⁴C] serine was added, and cultured at 33°C for 2 hours. After the cell was washed twice in 2 mL of cooled PBS, dissolved in 900 µL of sodium dodecyl sulfate (SDS), then, 800 µL and 20 µL of dissolved matter were used for determining lipid extraction and protein concentration. For the purpose of analyzing lipid labeled with [¹⁴C] serine, the lipid was separated by TLC by making acetic methyl/n-propanol/chloroform/methanol/0.25% KCl (25: 25: 25 10:9, volume ratio) development solvent. The radioactive lipid separated on the plate was detected and analyzed by an image analyzer. The results using [¹⁴C] serine are indicated in Fig. 4 (A), and the results using [¹⁴C] choline are indicated in Fig. 4(B).

### <Method of measuring enzyme activity>

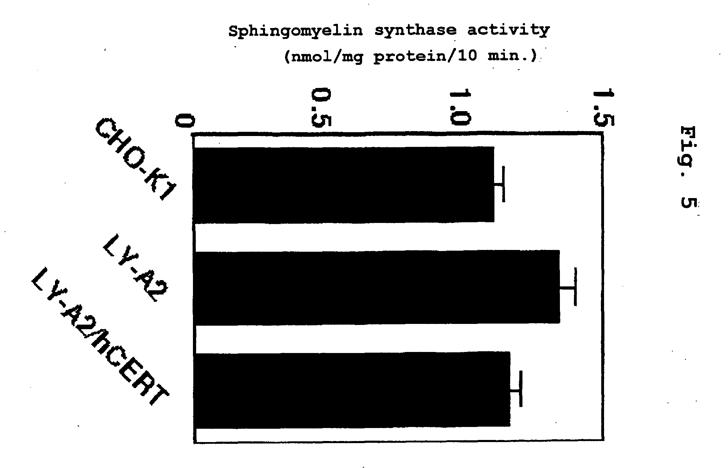

The assay of SM synthase activity was carried out using C₆-NBD-Cer as a substrate by a method of Hanada et al (Biochim. Biophys. Acta 1086, 151-156, 1991). However, the membrane fractions prepared respectively from CHO-K1 cell, LY-A2 cell and LY-A2/hCERT cell were used as an enzyme source. The results are indicated in Fig. 5.

### <Method of cell labeling using C₅-DMB-Cer and fluorescene microscopy observation>

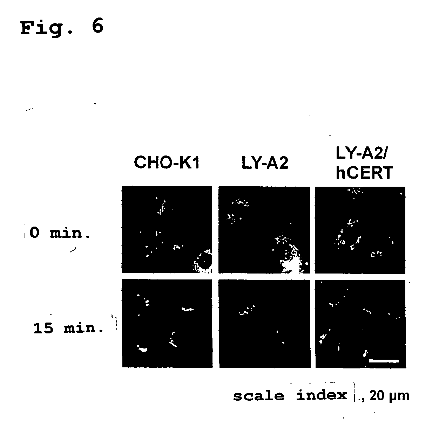

The present inventors have already made it clear that the activity of ATP dependent transport from the endoplasmic reticulum of natural ceramide (hereinafter, also referred to as ER) to Golgi apparatus can be qualitatively evaluated from the analysis of reallocation from ER of C₅-DMB-Cer which is a fluorescent ceramide analogue to Golgi apparatus (J. Cell Biol. 144, 673-685., 1999). As described in detail, the cell was exposed to C₅-DMB-Cer at 4°C for 30 minutes for the purpose of performing the pulse labeling of a variety of organelle membranes within a cell containing ER, after washing, it was traced at 33°C for 15 minutes by a fluorescene microscopy observation method. A method of performing cell labeling using C₅-DMB-Cer and a method of performing an observation by a fluorescence microscopy were carried out by a method of Yasuda et al (J. Biol. Chem. 276, 43994-44002., 2001) and a method of Fukasawa et al (J. Cell Biol. 144, 673-685., 1999). Specifically, after CHO-K1 cell, LY-A2 cell and LY-A2/hCERT cell grown on a coverslip made of glass were treated at 4°C for 30 minutes in F12 medium containing 1 micro-M C₅-DMB-Cer, it was washed three times in 1 mL of F12 medium. In the case where 1 mL of Nutridoma medium is added and not traced, immediately washed in PBS, on the other hand, in the case where it is traced, it was washed in PBS after it was cultured at 33°C for 15 minutes. Next, it was fixed at 4°C for 5 minutes in PBS solution of 0.125% glutaraldehyde. The sample was observed under the fluorescence microscopy immediately after it has been fixed and photographed with a digital CCD camera. The results are indicated in Fig.6.

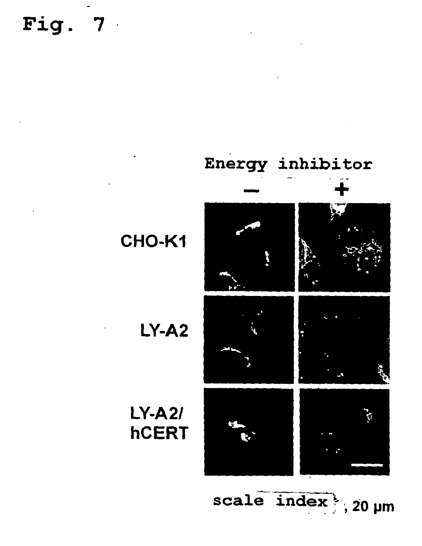

Furthermore, the present inventors have already made it clear that in the transport pathway through which ceramide transfers from ER to the place of SM synthesis, there are at least two pathways that is dependent on ATP and is not dependent on ATP, and in LY-A cell, ATP dependent transport pathway is deleted. For the purpose of examining whether or not ceramide transport pathway recovered by introducing hCERT is ATP dependent transport pathway, the influence of ATP depletion with respect to transfer within a cell of C₅-DMB-Cer was analyzed. It should be noted that in the case where ATP depletion conditions are used, after it was washed three times in 1 mL of F12 medium, pretreated at 33°C for 15 minutes in 1 mL of Nutridoma medium in which energy inhibitor (50 mM deoxyglucose and 5 mM sodium azide) has been added, it was C₅-DMB-Cer treated, washed in PBS, fixed similarly, observed and shot. Moreover, in the case where it is traced, after it was C₅-DMB-Cer treated, and further after it was cultured at 33°C for 15 minutes in the energy inhibitor added medium, it was washed in PBS, similarly fixed, observed and shot. The results are indicated in Fig. 7.

### <Influence of ceramide transport inhibitor>

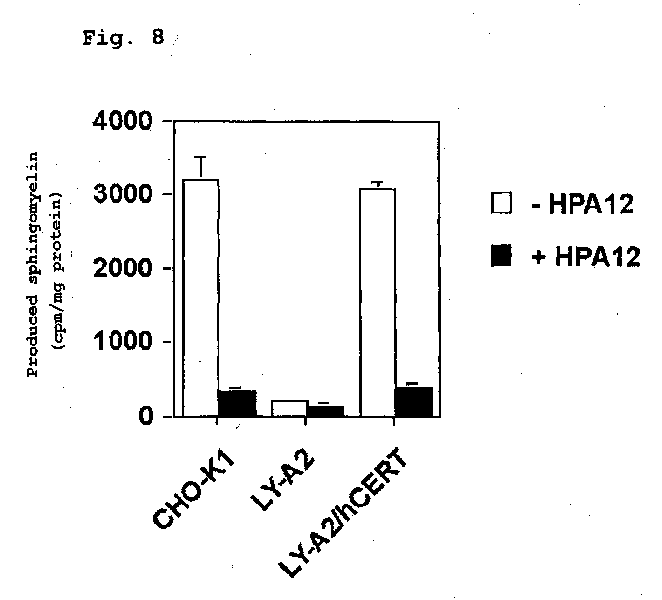

The present inventors have already found a selective inhibitor with respect to ATP dependent ceramide transport pathway (J. Biol. Chem. 276,43994-44002., 2001). For the purpose of examining whether or not ceramide transport pathway recovered by introducing hCERT is a transport pathway which is sensitive to (1R,3R) HPA-12, the analysis of the influence of (1R,3R) HPA-12 treatment with respect to SM biosynthesis was performed by performing a lipid metabolism labeling experiment using radioactive serine. The results are indicated in Fig. 8.

### <Recovery of ceramide transport function of LY-A cell by hCERT stable expression>

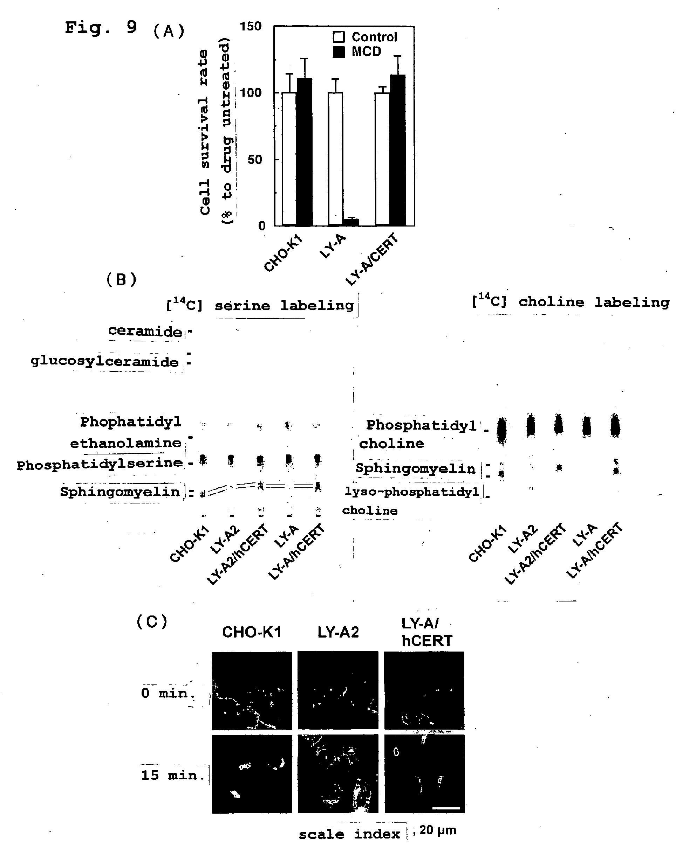

Also in the orignal LY-A cell to which retrovirus receptor has not been introduced, it has been ascertained as described below that ceramide transport function is recovered by hCERT expression. hCERT inserted pcDNA3.1/Neo plasmid was introduced into LY-A cell by performing lipofection, after G418 resistant cell was selected, transformed strain LY-A/hCERT was purified by a limited-dilution. Subsequently, the measurement of the sensitivity to MCD, metabolism labeling experiment of lipidusing [¹⁴C] serine and [¹⁴C] choline and cell labeling using C₅-DMB-Cer and fluorescene microscopy observation were similarly performed. The results are indicated in Fig. 9(A)-(C), respectively.

### <Evidence that deletion of LY-A cell is also complemented by hCERT_{L} cell>

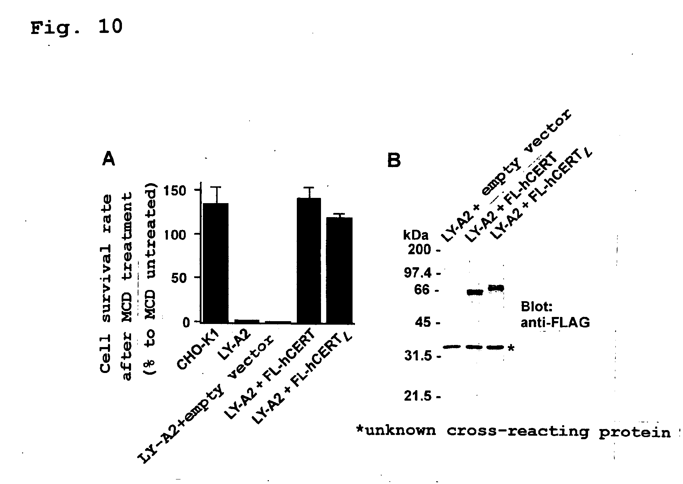

It has been validated whether or not CERT protein and CERT_{L} which is a large separate splicing type product having 26 amino acid residue complements the deletion of LY-A cell. LY-2A cell were infected with retroviruses for expressing CERT protein and CERT_{L} protein that FL sequence has been added to amino end, respectively. The MCD resistance of the obtained (LY-A2 +FL-hCERT) cell and ((LY-A2 +FL-hCERT_{L}) cell were measured by counting the number of survived cells in accordance with the method described in <Method of selecting MCD resistance recovery strain>. Moreover, the MCD resistance of (LY-A2+empty vector) cell, CHO-K1 cell and LY-A2 cell obtained by infecting LY-A2 cell using pLIB vector in which gene has not been inserted were also similarly measured. The results are indicated in fig. 10(A).

### <Western blot analyzing method using anti-FLAG antibody or anti-GFP antibody>

The method of preparing cell lysate and Western blot method were performed in accordance with a method of Hanada et al (J. Biol. Chem. 273, 337787-33794., 1998) and a emthod of Bejaoui et al (J. Clin. Invest. 110, 1301-1308., 2002). Specifically, (LY-A2+FL-hCERT) cell, (LY-A2+FL-hCERT_{L}) cell (LY-A2+empty vector) cell which have been washed in chilled PBS were collected by a method of scraping out, respectively, after it was suspended in HSEI buffer [in which 250 mM sucrose, 1 mM ethylendiaminetetraacetic acid (EDTA) and protease inhibitor cocktail have been added to 10 mM Hepes-NaOH buffer (pH 7.5)], the cells were lysed by performing supersonic treatment using an immersion supersonic generator. This cell lysate was subj ected to SDS-polyacrylamide gel electrophoresis, and transcrived to aPVDFmembrane. The immunological reaction protein were detected by performing a blocking treatment, the primary antibody treatment and its washing treatment, the secondary antibody treatment and its washing treatment, and then ECL treatment with respect to this blot membrane. For the purpose of detecting GFP or GFP fusion CERT protein, BD Living Colors™ A.v. monoclonal antibody was used as the primary antibody, respectively. As the secondary antibody, HRP binding goat anti-mouse immunoglobulin G was used. The results are indicated in Fig. 10(B).

### <cDNA cloning method of CERT protein derived from CHO-K1 cell>

cDNA of CERT protein derived from CHO-K1 cell was cloned by performing rapid amplification of cDNA ends (RACE) of cDNA ends using SMART RACE cDNA amplification kit manufactured by Clontech, Co., Ltd. Specifically, by making 5'-RACE Ready CHO cDNA prepared by utilizing the relevant kit template, and by making the primer #19 indicated in Table 1 gene specific primer, the PCR was carried out. At the time of carrying out this, Pyrobest DNA polymerase was used as a DNA polymerase. On the other hand, by making 3'-RACE Ready CHO cDNA template, and by making the primer #20 indicated in Table 1 gene specific primer, the PCR was carried out. The amplification products having about 1.2 kbp and about 1.4 kbp were obtained, respectively. After these products have been cloned in pCR-Blunt II-TOPO vector, the DNA sequence was determined. Considering overlapping region in the determined sequence, the sequence having the full length of cDNA, sequence 2473 bp was determined. Next, for the purpose of obtaining DNA containing the complete ORF of cCERT, the PCR was carried out by making CHO cell cDNA library template and by utilizing the primers #21 and #22 indicated in Table 1. The amplified DNA having about 1.9 kbp was treated by Eco RI and Xho I, the plasmid inserted into Eco RI-Xho I site of pBS vector was named as pBS/cCERT.

### <Northern blot analyzing method of CERT protein derived from CHO cell>

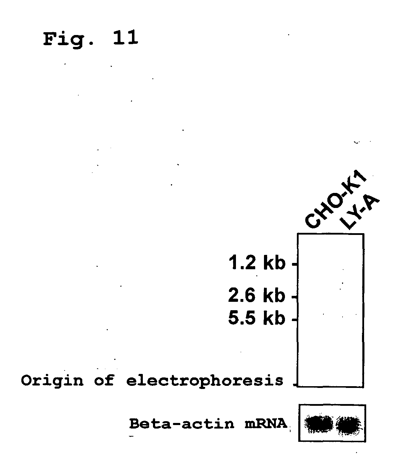

The total RNA was prepared from CHO-K1 cell and LY-A cell using isogen manufactured by Nippon Gene, Co., Ltd. Moreover, as a probe, a probe in which DNA having about 1.2 kbp obtained by the above-described cCERT 5'-RACE has been ³²P labeled using [α-³²P] dCTP and megaprime DNA labeling kit was used. Concerning with the washing conditions after the hybridization was carried out, it was performed under the stringent conditions. The analysis of radioactive pattern on the blot membrane was performed using BAS 2000 image analyzer. Moreover, the transcription membrane re-probing experiment for internal standard was performed by making a fragment that β-actin DNA fragment which has been commercially obtained from Wako Junyaku Kogyo, Co., Ltd. was ³²P labeled a probe. The results are indicated in Fig. 11.

### <Method of cloning of cCERT ORF derived from LY-A cell>

The total RNA was prepared from LY-A cell using isogen. Next, cDNA was prepared from this RNA using the superscript first chain synthesizing system. Next, the PCR was carried out by making the present cDNA a template and by utilizing the primers #21 and #22 indicated in Table 1. The amplified DNA having about 1.9 kbp was treated with Eco RI and Xho I, the plasmid inserted into Eco RI-Xho I site of pBS vector was named as pBS/cCERT (G67E). As described below, it was clarified that in cCERT derived from LY-A cell, a missense mutation that 67 glycine residue has been substituted by glutamic acid occurs, therefore, this protein was named as cCERT(G67E).

### <Method of adding FL sequence to carboxyl end of CERT derived from CHO-K1 cell or LY-A cell>

The PCR was carried out for the purpose of adding a FL sequence which is capable of admitting it with an anti-FLAG antibody by making pBS/cCERT template and by utilizing the primers #23 and #6 indicated in Table 1. DNA having about 300 bp obtained by treating this amplification product with Cla I and Xho I was purified. Then, DNA for coding CERT that FL sequence has added at carboxyl end was prepared by inserting the previously purified DNA having about 300 bp into the DNA having about 3.7 kbp obtained by treating pBS/cCERT and pBS/cCERT (G67E) with Cla I and Xho I and was cloned on pBS. After the sequence was ascertained, the cloned DNA was transferred into pLIB vector.

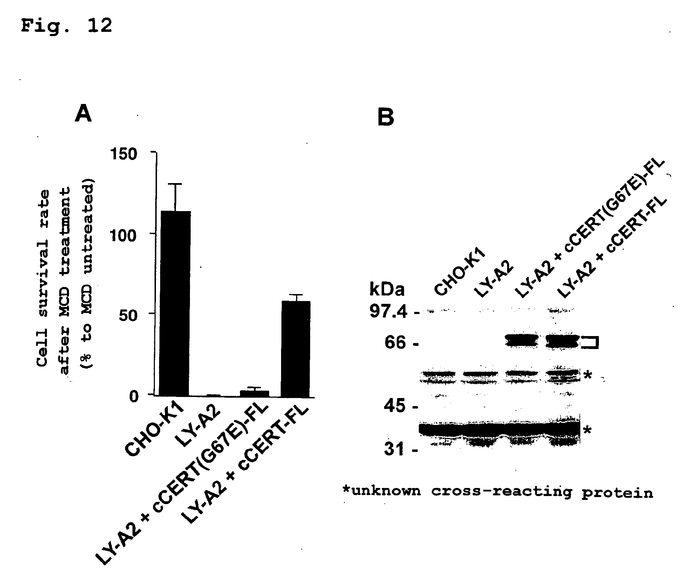

Moreover, whether or not the nature of LY-A2 cell is recovered to the wild type was analyzed by measuring the MCD resistance of (Ly-A2+cCERT-FL) cell and (LY-A2+cCERT(F67E) cell in which cDNA for coding FL sequence addition type (named as cCERT-FL and cCERT (G67E)-FL) of cCERT and cCERT(G67E) has been introduced into LY-A2 cell by means of counting the number of survived cells in accordance with the method described in <Method of selecting MCD resistance recovery strain >. The results are indicated in Fig. 12 (A) . Furthermore, Western blot analysis was performed similar to <Western blot analysis method using anti-FLAG antibody or anti-GFP antibody>with respect to cCERT-FL sequence and cCERT(G67E) -FL sequence. However, for the purpose of detecting FL sequence addition CERT protein, anti-FLAG M2 monoclonal antibody was used. The results are indicated in Fig. 12(B).

### <Method of preparing GFP fusion CERT expression plasmid>

Plasmid for expressing a fusion protein that GFP has been added at carboxyl end of hCERT, cCERT and cCERT (G67E) was prepared as the following.

The PCR was carried out by making pBS/hCERT, pBS/cCERT or pBS/cCERT (G67E) a template and by utilizing the primers #3 and #24 indicated in Table 1. After the amplified DNA having about 1.9 kbp was treated with Eco RI and Xho I and was inserted into Eco RI-Xho I site of pBS vector and cloned, the base sequence was admitted. Then, these cloned DNA was cut out by treating it with Eco RI and Xho I and inserted into Eco RI -Sal I site of pEGFP-N3 vector. The CERT-GFP fusion protein expression plasmids prepared in this way were systematically named as phCERT-GFP, pcCERT-GFP, pcCERT(G67E)-GFP and the like, respectively. It should be noted that the base sequence of the primers #3 and #25 are designed on the basis of DNA sequence of hCERT and is partially different from DNA sequence of the relevant portion of cCERT. However, since the amino acid sequences of the relevant portion are identified between hCERT and cCERT, and these primers were effective in PCR with respect to cCERT, these were used.

### <Method of expressing GFP and GFP fusion CERT in CHO cell>

pEGFP-N3, phCERT-GFP, pcCERT-GFP or pcCERT(G67E)-GFP plasmid was introduced into CHO-K1 cell using FuGene reagent. The obtained cells were named as (CHO-K1+pEGFP-N3) cell, (CHO-K1+phCERT-GFP) cell, (CHO-K1+pcCERT-GFP) cell, and (CHO-K1+pcCERT (G67E) -GFP) cell, respectively. After it has been cultured for 24 hours from the introduction initiation of DNA, the inoculation was performed again, and further after it has been cultured for 2 days, it was subjected to the observation of fluorescence or the preparation of cell lysate.

### <Method of analyzing co-localization of GFP or GFP fusion CERT expressedin (CHO-K1+pEGFP-N3) cell, (CHO-K1+pcCERT-GFP) cell, (CHO-K1+pcCERT (G67E)-GFP and Golgi apparatus localization marker)>

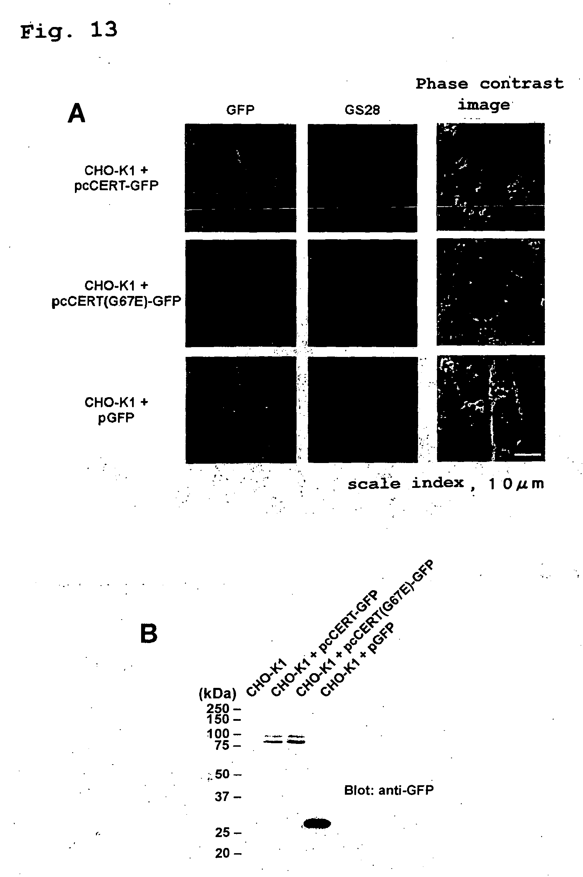

In (CHO-K1+pEGFP-N3) cell, (CHO-K1+phCERT-GFP) cell, (CHO-K1+pcCERT-GFP) cell, and (CHO-K1+pcCERT(G67E)-GFP) cell, in which CERT fusion GFP has been transiently expressed, indirect immunocyte staining using antibody with respect to GS28 protein locating in Golgi apparatus, and the distributions of GFP and GS28 within a cell were compared. The immunocytochemical method was performed in, accordance with a method of Yasuda et al (J. Biol. Chem. 278, 4176-4183., 2003). Specifically, after 3.7% formaldehyde fixation treatment, 0.1 M ammonia chloride treatment, 0.2% Triton X-100 treatment, blocking treatment, the primary antibody treatment and its washings, and the secondary antibody and its washings were performed with respect to a cell cultured on the coverslip, the cell was mounted on the slide glass, and the fluorescene microscopic observation was performed. However, a mouse anti-GS28 monoclonal antibody was used for the primary antibody, and alexa fluoro 594 goat anti-mouse immunoglobulin G was used for the secondary antibody. The results are indicated in Fig. 13(A).

### < Western blot method using anti-FLAG antibody or anti-GFP antibody>

A method of preparing a cell lysate and Western blot method were performed in accordance with a method of Hanada et al(J. Biol. Chem. 273, 33787-33794., 1998) and a method of Bejaoui et al(J. Clin. Invest. 110, 1301-1308., 2002). Specifically, cell which have been washed in cooled PBS were collected by a method of scraping out, after it was suspended in HSEI buffer [in which 250 mM scrose, 1 mM ethylendiaminetetraacetic acid(EDTA) and protease inhibitor cocktail have been added to 10 mM Hepes-NaOH buffer(pH 7.5)], the cells were lysed by performing supersonic treatment using an immersion supersonic generator. This cell lysate were subjected to SDS-polyacrylamide gel electrophoresis, and transcrived to a PVDF membrane. The immunological reaction protein were detected by performing a blocking treatment, the primary antibody treatment and its washing treatment, the secondary antibody treatment and its washing treatment, and then ECL treatment with respect to this transcription member. For the purpose of detecting GFP or GFP fusion CERT protein, BD Living Colors™ A. v. monoclonal antibody was used as the primary antibody, respectively. As the secondary antibody, HRP binding goat anti-mouse immunoglobulin G was used. The results are indicated in Fig. 13(B).

### <Preparation of vector for expressing hCERT protein and its related proteins that histidine tag sequence has added in E.coli>

For the purpose of expressing hCERT protein and its related proteins that histidine tag sequence has added in E.coli, the plasmid in which DNA fragment prepared and cloned in the paragraph from 0064 to 0066 of the specification of the present application has been transferred to multi-cloning site of pET28a(+) vector so that it is in-frame was prepared. Specifically, hCERT, hCERT_{L}, hCERTΔ PH, hCERTΔ MR, and hCERTΔ ST were collected by Eco RI-Xho I treatment, inserted it into Eco RI-Xho I site of pET-28a(+). On the other hand, PHhCERT, MRhCERT and SThCERT that have been cloned in pBS/nFL were collected by Hind III-Xho I treatment, and inserted into Hind III-Xho I site of pET-28a(+).

### <Method of purifying hCERT from recombinant E. coli>

The pET-28a(+) plasmid in which DNA for coding hCERT protein and its related protein (hCERT, hCERT_{L}, hCERTΔ PH, hCERT Δ MR, hCERT Δ ST, PHhCERT, MRhCERT and SThCERT) has been introduced into E. coli BL21 (DE3) strain by a heat shock method, and transformed as a resistant bacterium with respect to kanamycin. The transformed cell has been cultured at 37°C in the Luria broth medium until the cell turbidity reaches to 0.6 at the absorbance in the wavelength of 600 nm. At the time when the absorbance has reached to 0.6, IPTG (isopropyle-1-thio-beta-D-galactopyranoside) was added so that the final concentration becomes 250 µM, and further cultured at 25°C overnight. After the cultured liquid was centrifuged and suspended in a buffer for lysing the precipitated E.coli {25 mM Tris (pH7.4), 1% Triton X-100, 1 mM orthovanadic acid, 50 mM sodium fluoride, 5 mM sodium pyrophosphoric acid, 2.5 mM 2mercaptoethanol, 0.27Msucrose, and protease inhibitor mixture (Roche #1873580) one tablet/50 mL}, the bacterium was lysed using an immersion supersonic generator. This has been centrifuged at 100,000 g for one hour, and the supernatant fraction was used for purification. For purification, TALON (cobalt ion chelate resin) manufactured by Clontech, Co., Ltd. was used. The purification using TALON was performed in accordance with the manual. After the targeted protein eluted by 150 mM of imidazol was dialyzed overnight, substituted with a buffer consisting of 10 mM Tris (pH 7.4) and 250 mM sucrose, or a buffer consisting of 10 mM Tris (pH 7.4) and 150 mM of sodium chloride, it was stored at -80°C. As shown in Fig.1, it has been suggested that CERT has three domains of pleckstrin homology (PH) domain in about 100 amino acid region of amino end, steroidogenic acute regulatory protein-related lipid transfer (START) domain and a domain in the middle domain (MR) located between them. Then, the amino acid sequence of hCERT Δ PH protein, hCERTΔ MR protein, hCERTΔ ST protein whose respective domains have been solely deleted, and PHhCERT protein, MRhCERT prtein and SThCERT protein having solely the respective domains were also analyzed. Furthermore, a recombinant corresponding to hCERT_{L} protein having a structure that 26 amino acid has been inserted in the last of MR domain of hCERT was also prepared, and the amino acid sequence was analyzed. The sequence of hCERT protein is indicated as amino acid sequence of SEQ ID NO:1. the structures of hCERT Δ PH protein, hCERTΔ MR protein, hCERTΔ ST protein which are proteins solely deleted, and PHhCERT protein, MRhCERT protein and SThCERT protein having solely the respective domains are shown in Fig. 1. Moreover, the sequence of hCERT_{L} protein is indicated as amino acid sequence of SEQ ID NO:2. As described above, a protein used for a drug of the present invention was manufactured and used in the following Examples.

The followings were clarified in Figs. 2-13. As shown in Fig. 2, although LY-A2 has significantly acquired MCD resistance and lysenin sensitivity by virus particle infection derived from pLIB/hCERT, on the other hand, in the case of virus particle infection derived from pLIB vector, such a change did not occur at all. From the results, it was indicated that the reactivity with respect to MCD and lysenin of LY-A2 is changed into the nature similar to the wild type by the expression of hCERT.

As shown in Fig. 3(A), the sensitivity to MCD and lysenin of LY-A2/hCERT cell which is a purified cell was approximately the same level as the sensitivity seen in the wild type CHO-K1 cell. Furthermore, as shown in Fig. 3(B), when the respective phospholipids contained in a cell was chemically quantified, in LY-A2/hCERT cell, the SM content was also recovered to the wild type level. Moreover as shown in Fig. 4(A), when a lipid metabolism labeling experiment using radioactive serine was performed, the SM biosynthesis speed was also recovered to the wild type level in LY-A2/hCERT cell. From these results, it has been clarified that the lowering of SM biosynthesis in LY-A2 is approximately completely recovered by the stable expression of hCERT to the wild type level.

### <Evidence that SM synthesis recovery in LY-A2 cell is not due to increase of SM synthesizing enzyme activity or PC biosynthesis>

As shown in Fig.4 (B), SM is synthesized by transfer of phosphocholine from phosphatidylcholine (PC) to ceramide, the relevant reaction is catalyzed by SM synthase. Also in a lipid metabolism labeling experiment using radioactive choline, the SM biosynthesizing rate of LY-A2/hCERT cell was recovered to the wild type level, and the PC synthesizing rate was also at the wild type level. As shown in Fig. 5, concerning with the activity of the SM synthase, there was no difference among LY-A2/hCERT, LY-A2 and CHO-K1 cells. From these results, it has been indicated that the SM synthesis recovery in LY-A2 cell does not occur due to the increase of SM synthase activity and PC synthesis.

### <Re-allocation from ER of C₅-DMB-Cer to Golgi apparatus in a living cell>

As shown in Fig. 6, LY-A2/hCERT, LY-A2 and CHO-K1 cells indicate the pattern of DMB fluorescence substantially within the same cell before the trace, and it has been distributed approximately in a uniform state in the whole of the organic membrane within a cell. On the other hand, when the labeling cell was traced, the accumulation of DMB fluorescene to Golgi apparatus region in LY-A2 cell was apparently comparing to CHO-K1 cell, however, the accumulation comparable to CHO-K1 cell was observed in LY-A2/hCERT.

As shown in Fig. 7, under the conditions where ATP within a cell was depleted by performing the treatment of an energy inhibitor (50 mM deoxy-D-glyucose and 5 mM NaN₃), the transfer of DMB fluorescence from ER to Golgi apparatus region was inhibited also in LY-A2 cell similar to that in CHO-K1 cell. From these results, it was clarified that ATP dependent ceramide transport between ER and Golgi apparatus which is deleted in LY-A2 cell is approximately completely recovered by the stable expression of hCERT.

As shown in Fig.8, 1 µM of HPA-12 has significantly inhibited the SM synthesis also in LY-A2 cell similarly in CHO-K1 cell. In LY-A2 cell where (1R,3R) HPA-12-sensitive transport pathway has been impaired, even in the case where (1R,3R) HPA-12 has not been treated, the SM biosynthesis was lowered, even if the present drug treatment was performed, another additional inhibition was not seen. From these results, it was clarified that ceramide transport pathway recovered by the introduction of hCERT is (1R,3R) HPA-12-sensitive pathway.

As shown in Fig. 9, in all of the indexes such as reactivity with respect to MCD, SM biosynthesis, C₅-DMB-Cer transfer from ER to Golgi apparatus region, LY-A/hCERT cell was recovered to the nature of the wild type.

As shown in Fig. 10(A), it has been validated whether or not another splicing type product/hCERT_{L} which is 26 amino acid residue larger than hCERT protein complements the deletion of LY-A cell. In the case where LY-2A cell were infected with retroviruses for expressing hCERT (FL-hCERT) or hCERT_{L}(FL-hCERT_{L}) that FL sequence has been added to amino end, it significantly acquired the MCD resistance, on the other hand, in the case of virus particle infection derived from emptyvector, such a change was not occurred. As shown in Fig. 10(B), according to Western blot analysis with respect to FL sequence, the introduction expression efficiencies of FL-hCERT and FL-hCERT_{L} were approximately equal. From these results, it has been clarified that hCERT_{L} has an ability to complement the deletion in LY-A cell similarly to hCERT.

### <Sequence analysis of CERT cDNA derived from CHO-K1 cell>

The sequence of the full length cDNA with respect to CERT mRNA derived from CHO-K1 cell was determined by RACE or the like. The sequence having the full length of 2473 bp contained ORF having 1794 bp for coding the product (cCERT) of 598 amino acid (Sequence tables 3 and 7) . CERT is 598 amino acid in all of human, mouse and Chinese hamster, and the homology of about 90% exists at DNA sequence level and the homology of about 98 % exists between cCERT and hCERT. The results indicate the strong conservation of CERT among mammals. Moreover, cDNA library derived from CHO cell, ORF for coding protein homologous to hCERT_{L} (named as cCERT_{L}) was also amplified by a PCR (Sequence tables 4 and 8). cCERT_{L} is a product larger than cCERT by 26 amino acid similar to the relationship between hCERT_{L} and hCERT.

### <Northern blot analysis of CHO cell CERT>

As shown in Fig. 11, for the purpose of examining whether or not there is a difference in CERT mRNA expression between CHO-K1 cell and LY-A cell, Northern blot analysis was performed. RNA having three different molecular weights (about 1.2, 2.6, 5. 5 kilo base) hybridized to CERT probe was detected, there was no significant difference between CHO-K1 cell and LY-A cell at any level of expression. The full length of CERT cDNA cloned from CHO-K1 cell was 2473 bp, therefore, it is considered that RNA having about 2.6 kilo base detected in Northern blot analysis is probably mRNA corresponding to the full length of CERT cDNA. The other RNAs having 1.2 and 5.5 kilo base may be splice isoform or immature type CERT mRNA. For the purpose of obtaining internal control, a re-hybridization was performed using β actin DNA as a probe, and it has been also admitted that actin mRNA level is equal between both cell RNA samples.

### <Sequence analysis of CERT cDNA derived from LY-A·cell>

As indicated in the foregoing paragraph, it has been found that also in LY-A cell, CERT mRNA was expressed. Then, CERT cDNA was cloned from LY-A cell by reverse transcription PCR. It has been clarified that CERT cDNA of LY-A cell has the sole mutation comparing to sequence derived from CHO-K1 cell. This mutation is single base substitution in which 67th codon of ORF is changed from GGA to GAA, as a result of this, it was a missense mutation in which 67 glycine residue of cCERT protein is substituted by glutamic acid residue. Then, this ORF product was named as cCERT (G67E). It should be noted that in the present experiment, two independent PCR experiments were carried out and the identical sequence was obtained. Hence, the missense mutation previously described is not an artificial mutation by PCR.

### <cDNA introduction expression effect of cCERT or cCERT(G67E) in LY-A2 cell>

As shown in Fig. 12(A), in the case where cCERT-FL cDNA has been introduced into LY-A2, MCD resistance recovery clearly occurred, however, in the case where its G67E variant has been introduced, such a recovery did not occur. Even in the case where cDNA of cCERT without adding FL sequence and cCERT(G67E) has been introduced, similar results were obtained (data not indicated). Moreover, as shown in Fig. 12(B), it has been indicated that the expression levels of cCERT-FL and cCERT (G67E) -FL in Western blot analysis with respect to FL sequence are approximately the same with each other. Hence, the possibility that since the expression level of cCERT (G67E) is lower than that of cCERT when these are compared has been denied. From these results, since the defect of LY-A cell is complemented by expression of wild type cCERT, it has been clarified that the relevant complementary function of cCERT is damaged by G67E mutation.

It should be noted that in the results of Western blot analysis shown in Fig. 10(B) and Fig. 12(B), it is considered that the reason why CERT is observed as a double band is caused by phosphorylation of the present protein. Actually, when a dephosphorylation treatment has been performed, the band indicating a higher molecular weight out of the double band was deleted.

### <Influence of G67E with respect to CERT distribution within a cell>

As shown in Fig. 13(A), in the case where cCERT-GFP is expressed, its green fluorescence distributes in an approximately uniform state also in the cytoplasm, and clearly concentrated in the perinuclear region. Since this perinuclear region corresponds to GS28 localization region, it is considered that it is Golgi apparatus. On the other hand, in the case where cCERT(G67E)-GFP has been expressed, the green fluorescence distributed in an approximately uniform state in the cytoplasm, and was not selectively concentrated in GS28 localization region. Moreover, in the case where GFP not fused with CERT has been expressed, a large amount of the green fluorescence distributed also in nucleus as well as in the cytoplasm. It should be noted that from the Western blot analysis using an anti-GFP antibody shown in Fig. 13(B), cCERT-GFP and cCERT(G67E)-GFP have been expressed as a product having the expected molecular weight, and decomposition product reacting with the GFP antibody was not detected. Hence, the GFP fluorescence distribution observed at the time of GFP fusion protein expressing reflects cCERT-GFP or cCERT(G67E) -GFP distribution itself. From these results, it has been clarified that CERT distributes mainly in cytoplasm and Golgi apparatus, the association ability of Golgi apparatus of CERT is damaged by G67E mutation, and G67E mutation does not have an influence on the nature that CERT is excluded from nucleus.

When hCERT cDNA was introduced into LY-A strain, the SM synthesis was recovered to the wild type level. Moreover, it has been also clarified by an assay using fluorescent ceramide analogue that ceramide transfer from endoplasmic reticulum to Golgi apparatus was recovered. hCERT_{L} corresponding to a separate splicing type also has made LY-A strain recovered. Hence CERT protein is a factor involving in ceramide selective transport within a cell.

From the results described above, it has been clarified that (1) CERT protein has been selected from cDNA library by a method of searching the function recovery of LY-A cell having the default involving in ceramide transport, (2) CERT protein complements all of the known defects of LY-A cell, (3) in LY-A cell, missense mutation is occurring in an endogenous gene for CERT protein, (4) CERT protein having the mutation occurring in LY-A cell damages the defect-complementing ability. Therefore, from these results, it has been clarified for the first time that the mutation in the gene for coding the relevant CERT protein is a causal mutation of the defect of LY-A cell, and CERT is a factor specifically involving in ceramide selective transport within a cell.

### EXAMPLE 1

### <hCERT protein, hCERT_{L} protein and its recombinant protein>

Next, whether or not hCERT protein and its recombinant protein (hCERT protein, hCERT_{L} protein, hCERTΔ PH protein, hCERT Δ MR protein, and hCERTΔ STprotein, and further, PHhCERT protein, MRhCERT protein and SThCERT protein) indicates the activity for releasing ceramide from lipid membrane was considered. In this assay, hCERT protein and its recombinant protein (hCERT protein, hCERT_{L} protein, hCERTΔ PH protein, hCERTΔ MR protein, and hCERT Δ ST protein, and further, PHhCERT protein, MRhCERT protein and SThCERT protein) which were expressed by E.coli described above, used in <Preparation of vector for expressing hCERT protein and its related protein to which histidine tag sequence has been added by E. coli > and <Method of purifying hCERT from recombinant E. coli> were purified and used.

### <Method of detecting ceramide release promotion activity from membrane>

Hereinafter, the standard method will be described in detail, however, even if the described conditions are changed, a method of detecting ceramide release promotion activity having means for separating releasing ceramide by a centrifugation principally belongs to the present invention. The lipid membrane containing ceramide was prepared so that it contains 12.5 nCi (225 pmol) per sample of [palmitoyl-1-¹⁴C] N-palmitoyl-D-ethyro-sphigosine (hereinafter, may be referred to as ¹⁴C-ceramide) on the basis of the mixed lipid consisting of phosphatidylcholine and phosphatidylethanolamine derived from egg yolk at the ratio of 4:1 and its concentration becomes 2.5 mg/mL. This lipid membrane is required at the rate of 20 µL per one sample of the activity measurement. After the amount of lipid required for activity measurement was dispensed in Eppendorf tube, it was dried by spraying nitrogen gas. After the buffer 1 [20 mM Hepes-NaOH buffer (pH7.4) to which 50 mM NaCl and 1 mM EDTA have been added] was added to the dried lipid membrane so that the concentration becomes 2.5 mg /mL, the supersonic treatment was gently performed using bath type supersonic generator [Model 2210 manufactured by Branson, Co., Ltd.]. The supersonic treatment was performed at 25°C, and the procedure that the supersonic treatment for 3 minutes, the vortex for 30 seconds and the supersonic treatment for 3 minutes were performed in this order. The lipid membrane prepared in this way was used in ceramide release experiment. The ceramide release reaction for lipid membrane and its detection were performed as the followings.

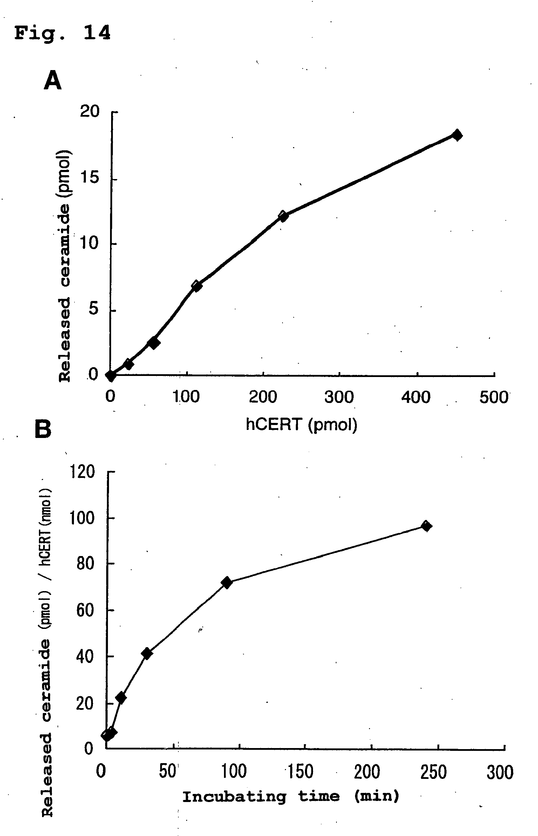

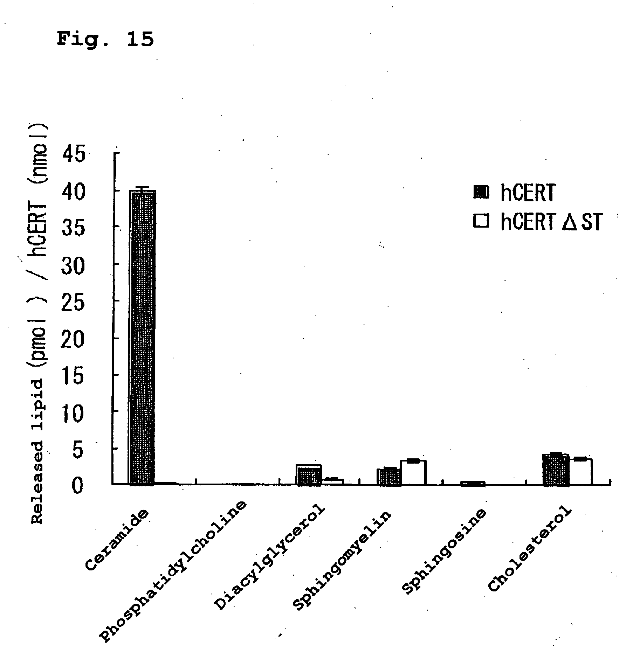

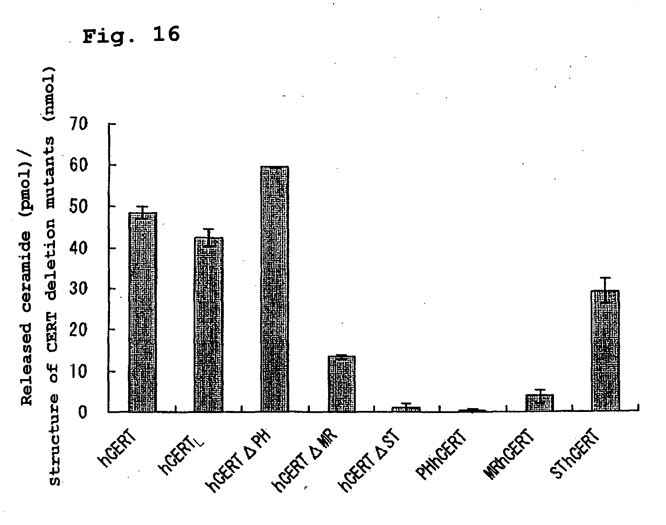

hCERT protein or its recombinant protein described above as a protein sample to be targeted (under the standard conditions, the amount of protein corresponding to 450 picomoles, which is 2-fold molar equivalent amount of ceramide contained in the donating membrane was used) were messed up to 30 µL using the buffer 2 [50 mM Hepes-NaOH buffer (pH7.4) to which 100 mM NaCl and 0.5 mM EDTA have been added] . Here, the reaction was initiated by adding 20 µL of lipid membrane containing ceramide. The final concentration of phospholipids became 1 mg/mL, and ceramide was contained at the ratio of about 0.3% comparing to the total phospholipid amount. After the mixture of these has been incubated at 37°C for 30 minutes, it was centrifuged at 50,000 xg for 30 minutes (centrifuging machine: himac CS120EX manufactured by HITACHI Engineering Machine, Co., Ltd.: centrifuging rotor: RP100AT3-200; centrifugation tube, 0.23PC tube) and the lipid membrane was precipitated. In the case where hCERT protein and its recombinant protein which has been purified from the recombinant E.coli is used, most of the protein remains in the supernatant under these centrifugation conditions, therefore, when ¹⁴C-ceramide binds to hCERT protein, it releases from the lipid membrane and transfers to the supernatant fraction. The activity for promoting ceramide release with hCERT was calculated by measuring the radioactive activity of ¹⁴C in the supernatant fraction using a liquid scintillation counter. The results of measurement of the activity for promoting ceramide release using hCERT protein are indicated in Figs. 14(A) and (B) . The substrate from membrane vesicle in a releasing reaction assay was changed from ceramide to a variety of other lipids, and the specificity of the reaction was examined. At this time, the difference between hCERT and START domain deletion hCERT was made the index of the lipid drawing activityvia START domain. The results are indicated in Fig. 15. Furthermore, the measurement results of ceramide release activity using hCERT protein, hCERT_{L} protein, hCERTΔ PH protein, hCERTΔ MR protein, and hCERTΔ ST protein, and further, PHhCERT protein, MRhCERT protein and SThCERT protein are indicated in Fig. 16.

The activity for promoting ceramide release was measured using the purified recombinant hCERT. When phospholipid multilamellar vesicle containing a trace of ceramide was centrifuged, ceramide was almost completely precipitated accompanying with phospholipid vesicle. However, in the case where hCERT co-exists, ceramide released from the membrane and allocated to the supernatant fraction accompanying with hCERT while phospholipid multilamellar vesicle was completely precipitated. Specifically, as it is understood from Figs. 14 (A) and (B), the ceramide release from phospholipid membrane was detected depending on the amount of hCERT and the length of incubating time. On the other hand, in the case where hCERT has not been added, ceramide release was approximately zero. From the results, it has been clarified that hCERT has the activity for promoting ceramide release from the membrane.

Up to now, as a lipid that protein having START domain except for CERT admits, phosphatidylcholine and cholesterol are known. However, as shown in Fig. 15, CERT protein did not indicate a significant releasing reaction with respect to diacylglycerol, cholesterol, phophatidylcholine, sphingomyelin, sphingosine. Moreover, diacylglycerol releasing promotion which is similar to ceramide from the viewpoint of structure was about 5% of the activity with respect to ceramide. From the results, it has been clarified that CERT has the activity for specifically releasing ceramide from the membrane.

As shown in Fig. 16, a variety of deleted variants of hCERT were prepared and purified as a recombinant, and the activity for promoting ceramide release that the respective domains have has been considered. Moreover, hCERT_{L} has also indicated that the activity is similar to that of hCERT. The deletion of PH domain did not have an influence on the activity for promoting ceramide release, the deletion of MR domain has lowered the activity in some degrees. On the other hand, this ceramide release reaction did not occur when START domain was deleted from hCERT protein, on the other hand, even the SThCERT protein having only START domain indicated the activity. From the results, it has been clarified that ceramide release from the membrane due to hCERT occurs via START domain. From the results described above, it has been clarified that CERT and CERT_{L} have the activity for specifically releasing ceramide from the lipid membrane via its START domain.

From the results described above, it has been clarified that CERT and CERT_{L} have the activity for specifically releasing ceramide from the lipid membrane via its START domain.

### EXAMPLE 2

### <Method of measuring activity for promoting ceramide intermembrane transfer>

Subsequently, as described in detail below, whether or not hCERT protein and recombinant protein promotes the transfer between ceramide lipid membrane was considered, the purified hCERT protein and its recombinant protein were used in this assay were used. The present method was constructed on the basis of the principle of method of measuring the activity for promoting the intermembrane tranfer of phosphatidylinositol by phosphatidylinositol transer protein already reported by Kasper et al (Biochim. Biophys. Acta 664, 22-32., 1981) so that it becomes a new method of measuring the activity for promoting the ceramide intermembrane transfer. For the purpose of measuring the activity for promoting the intermembrane transfer, a donating membrane in which radioactive ceramide has been contained and a receiving membrane not containing ceramide were prepared. For the purpose of separating the lipid membrane, lactocylceramide was contained in the donating membrane. After the donating membrane and receiving membrane have been incubated with the protein sample to be targeted, castor seed lectin was added. Ceramide intermembrane-transferred can be quantified by measuring the radioactive activity of receptor membrane which has not been precipitated by a low speed centrifugation. Hereinafter, the standard method will be described in detail, however, principally, all of the method of introducing selective recognition marker into either of ceramide donating membrane or receiving membrane detecting the activity for promoting the intermembrane transfer having means for separating ceramide donating membrane and receiving membrane by utilizing the marker are belonged to the present invention.

The donating membrane was prepared by adding 12.5 nCi per one sample and 125 nCi of [2-palmitoyl-9, 10-³H (N)] L-α-dipalmitoyl phosphatidylchloine (hereinafter, may be referred to as ³H-DPPC) so that the total lipid concentration becomes 1.77 mg/mL. The substance amount of ceramide to be targeted was achieved by adding the non-radioactive ceramide to ¹⁴C-ceramide. This lipid membrane is required at the rate of 20 µL per one sample of the activity measurement. After the amount of lipid required for activity measurement was dispensed in polypropylene microtube, it was dried by spraying nitrogen gas. After the buffer 1 [20 mM Hepes-NaOH buffer (pH7.4) to which 50 mM NaCl and 1 mM EDTA have been added] was added to the dried lipid membrane so that the concentration becomes 1.77 mg/mL, the supersonic treatment was performed using an immersion supersonic generator (for example, UP50H manufactured by KUBOTA, Co., Ltd.). The supersonic treatment was performed at 25°C for 10 minutes in water bath. Even if the donating membrane prepared in this way is centrifuged under the conditions of 4°C, 12,000 x g and 30 minutes (centrifuging machine: MRX-150 manufactured TOMY, Co., Ltd.; centrifugation tube, 1.5-ml micro-test-tube manufactured by Eppendorf, Co., Ltd.), 90% or more of the lipid membrane is collected in the supernatant without precipitation.

The lipid membrane remained in the supernatant and the lipid membrane before the centrifugation was measured by a liquid scintillation counter, and it has been admitted that the radioactive activity of ³H is not changed between both in the respective experiments (normally, 90% or more was collected in the supernatant) . Moreover, it has been admitted that the ratio of precipitation/non-precipitation found in radioactive activity of ³H indicated approximately the same value as the value calculated by phosphorus quantitative determination and it is possible that the allocation amount of the total donating membrane from the allocation amount of ³H-DPPC is estimated.

The receiving membrane, which is a mixed lipid consisting of phosphatidylcholine and phosphatidylethanolamine derived from egg yolk at the ratio of 4/1, was prepared so that the total lipid concentration becomes 5.33 mg /mL. This lipid membrane is required at the rate of 60 µL per one sample for activity measurement. After the amount of lipid required for measuring the activity has been dispensed in a 1.5-mL micro-test-tube (manufactured Eppendorf, Co., Ltd.), nitrogen gas was sprayed to it and dried. After the buffer 1 has been added the dried lipid membrane so that the concentration becomes 5.33 mg /mL, the supersonic treatment was performed using an immersion supersonic generator (for example, UP50H manufactured by KUBOTA, Co., Ltd.). The conditions of the supersonic treatment, subsequent centrifuging conditions and the like were performed similar to the preparation of the donating membrane.

The detection of the activity for promoting ceramide intermembrane transfer was performed as the followings.

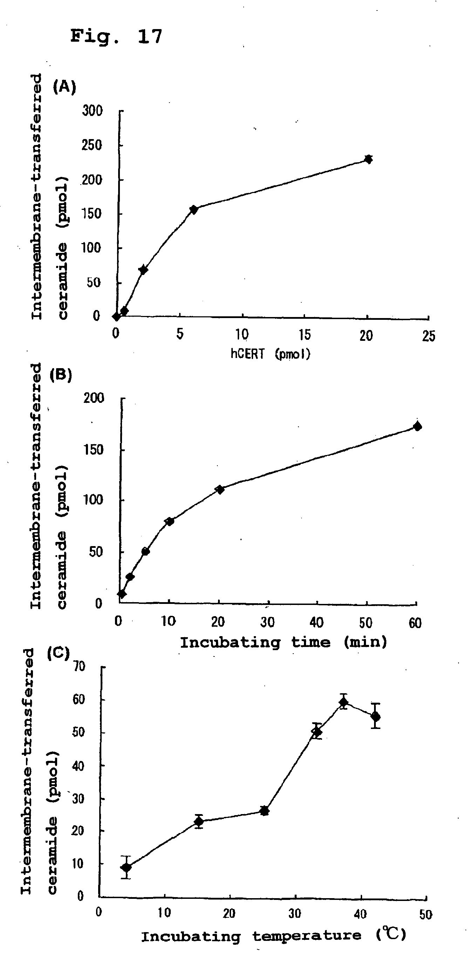

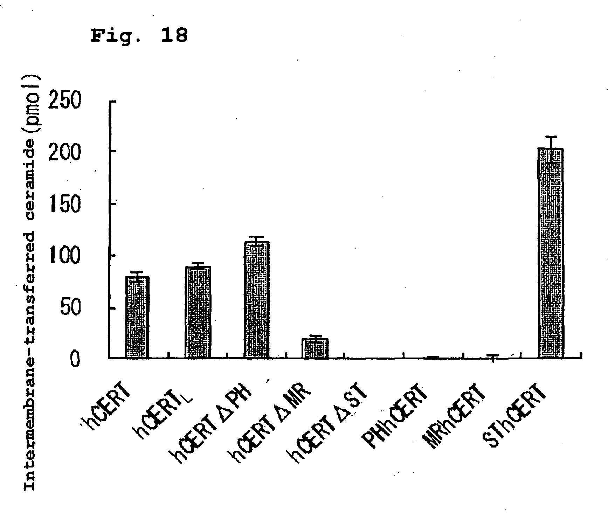

hCERT protein, hCERT_{L} protein, hCERTΔ PH protein, hCERT Δ MR protein, or hCERTΔ ST protein, or PHhCERT protein, MRhCERT protein or SThCERT protein (under the standard conditions, a protein corresponding to 2 picomoles corresponding to 0.4% molar equivalent amount of ceramide in the donating membrane was used) which is a protein sample to be targeted, and 60 µL of receiving membrane was messed up to 80 µL using the buffer 1 in 1.5-mL micro-test-tube (manufactured by Eppendorf , Co., Ltd.). Here, the reaction was initiated by adding 20 µL of donating membrane, and it has been incubated at 37°C for 15 minutes. The reaction was terminated by adding 30 µL of castor seed lectin and agitating by means of pipetting. For the purpose of sufficiently forming an aggregate, after it was cooled further for 15 minutes in ice bath, it was centrifuged under the conditions of 4°C, 12,000 xg, 3 minutes. The supernatant was collected by a pipette and the precipitated donating membrane was dissolved in 130 µL of 0.1% SDS. The activity for promoting ceramide intermembrane transfer that the sample has was measured by measuring the radioactive activity of ¹⁴C of the supernatant and the donating membrane. Even in the case where the sample is not contained, ceramide extremely slightly intermembrane-transfers, therefore, it is subtracted as background by free diffusion. It has been admitted in each experiment that the donating membrane and receiving membrane are selectively separated by admitting that the most part of the radioactive activity derived from ³H-DPPC that has been added to the donating membrane (usually, 90% or more) is precipitating accompanying with the donating membrane. The results that the activity for promoting ceramide intermembrane transfer are indicated in Figs. 17(A), (B) and (C) using the purified recombinant hCERT. Furthermore, the results of measuring the activity for promoting ceramide intermembrane transfer due to hCERT protein, hCERT_{L} protein, hCERTΔ PH protein, hCERTΔ MR protein, and hCERTΔ ST protein, and PHhCERT protein, MRhCERT protein and SThCERT protein are indicated in Fig. 18.

### <Method of quantifying protein>

The protein was quantified using BCA assay reagent of Pierce, Co., Ltd. BSA was used for the reference.

As shown in Figs. 17(A), (B) and (C), the transfer intermembrane of ceramide was detected depending on the amount of hCERT, the length of incubation time and incubation temperature. On the other hand, ceramide intermembrane transfer was approximately zero in the case where hCERT has not been added. In the case where ceramide donating membrane vesicle containing a trace of radioactive ceramide and a receiving membrane vesicle not containing ceramide were mixed and incubated, ceramide did not transfer to the receiving membrane vesicle at all even at the time when one hour has passed. However, when hCERT co-exists, ceramide transfer occurred. Ceramide close to 50% for 10 minutes was transferred to the receiving membrane vesicle by the added amount of CERT. It should be noted that the fusion between the donating membrane vesicle and receiving membrane vesicle did not occur regardless of hCERT. From the results, it has been clarified that hCERT has the activity for promoting ceramide intermembrane transfer.

As shown in Fig. 18, concerning with a variety of deletion variants of hCERT, the activity for promoting ceramide intermembrane transfer that the respective domains has been considered using a recombinant. The deletion of PH domain did not have an influence on the activity for promoting ceramide release and, the deletion of MR domain has lowered the activity to some degree. On the other hand, START domain was deleted, the activity was completely deleted. Furthermore, a high activity was detected even only in START domain, the activity was not detected at all only in the other domains. It should be noted that hCERT_{L} has also indicated that the activity is similar to that of hCERT. Hence, it has been clarified that CERT and CERT_{L} have the activity for specifically releasing ceramide from the lipid membrane via its START domain.

### INDUSTRIAL APPLICABILITY

The present invention can provide a novel drug for promoting ceramide transport. Moreover, the present invention can provide base sequence used for producing a drug of the present invention. Moreover, the present invention can provide a method of measuring the activity for promoting a novel ceramide release. Furthermore, the present invention can provide a method of measuring the activity for promoting a novel ceramide intermembrane transfer.

### Sequence table free text

SEQ ID NOs: 1-4 indicate an amino acid sequence of hCERT protein, an amino acid sequence of hCERT_{L}, an amino acid sequence of cCERT protein, and an amino acid sequence of cCERT_{L} protein, respectively. SEQ ID NOs: 5-8 indicate ORF sequence of hCERT mRNA, ORF sequence of hCERT_{L} mRNA, the full length cDNA sequence of cCERT mRNA and DNA sequence of cCERT_{L} ORF. SEQ ID NOs: 9-32 indicate sequences of primers.

## Claims

1. A drug for promoting ceramide transport comprising hCERT protein having an amino acid sequence of SEQ ID NO: 1, hCERT_{L} protein having an amino acid sequence of SEQ ID NO: 2, cCERT protein having an amino acid sequence of SEQ ID NO:3, cCERT_{L} protein having an amino acid sequence of SEQ ID NO: 4, or their recombinant proteins as an effective component.

2. The drug of claim 1, said drug being a drug used as an antitumor agent, an anti-inflammatory agent, an organoregenesis agent, an anti-infective agent, or a distribution promoting agent used for cosmetics.

3. The drug of claim 1, said drug being a drug used for detecting a drug for inhibiting ceramide transfer.

4. The drug for promoting ceramide transport of claim 1, said drug whose effective component is a recombinant protein consisting of 370 residue to 598 residue of an amino acid sequence of SEQ ID NO:1 or 3, or 397 residue to 624 residue of an amino acid sequence of SEQ ID NO. 2 or 4.

5. A base sequence of SEQ ID NOs: 5, 6, 7 or 8 or its recombinant base sequence, said base sequence being used for producing a drug of claim 1.

6. The base sequence of claim 5, wherein a recombinant base sequence consists of 1108 base pair to 1794 base pair of the base sequence of SEQ ID NO:5, 1189 base pair to 1872 base pair of the base sequence of SEQ ID NO:6, 1539 base pair to 2225 base pair of the base sequence of SEQ ID NO: 7, or 1189 base pair to 1872 base pair of the base sequence of SEQ ID NO: 8.

7. A method of measuring an activity for promoting ceramide release, comprising: an incubation process for incubating a mixture obtained by mixing a lipid membrane containing ceramide and a drug for promoting ceramide release, a separating process for obtaining a supernatant from the mixture after it has been incubated by separating using centrifugation, and a quantification process for quantifying ceramide contained in the obtained supernatant.

8. The method of measuring the activity for promoting ceramide release of claim 7, wherein a lipid membrane containing the ceramide is prepared by adding ceramide to the mixed lipid of phosphatidylcholine and phosphatidylethanolamine.

9. The method of measuring the activity for promoting ceramide release of claim 7, wherein a lipid membrane containing the ceramide is subjected to a supersonic treatment.

10. The method of measuring the activity for promoting ceramide release of claim 7, wherein a ceramide added to the lipid membrane containing the ceramide is a creamide radioactively labeled.

11. A method of measuring an activity for promoting ceramide intermembrane transfer, comprises: an incubating process for mixing a receiving membrane, a drug for promoting ceramide transfer, a donating membrane and incubating the obtained mixture, a separating process for separating the receiving membrane and the donating membrane by being subjected to a centrifugation after a membrane aggregating agent is selectively added to the mixture obtained in the incubating process, and a quantification process for quantifying ceramide contained by the separated receiving membrane and the donating membrane, respectively.

12. The method of measuring the activity for promoting ceramide intermembrane transfer of claim 11, wherein the receiving membrane is prepared by the mixed lipid between phosphatidylcholine and phosphatidylethanolamine.

13. The method of measuring the activity for promoting ceramide intermembrane transfer of claim 11 or 12, wherein a donating membrane containing the ceramide is prepared by the mixed lipid containing phosphatidylcholine, phosphatidylethanol, lactocylceramide and ceramide.

14. The method of measuring the activity for promoting ceramide intermembrane transfer of claim 11, wherein a ceramide added to the donating membrane containing the ceramide is a ceramide radioactively labeled.

15. The method of measuring the activity for promoting ceramide intermembrane transfer of any one of claims 10-14, wherein the selective membrane aggregating agent is a castor seed lectin.


SEQUENCE LISTING

<110>  Japan Science and Technology Corporation
       manager of Natinal Insutitute of Infectious Diseases

<120>  Medicine for promoting ceramide transportation, sequence for
manufacturing the medicine, method for measuring the promoting activity for
ceramide isolation, and method for measuring the promoting activity for ceramide
migration between membranes

<130>  P000

<160>  32

<170>  PatentIn version 3.1

<210>  1
<211>  598
<212>  PRT
<213>  HeLa cell

<400>  1

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp

180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val
        355                 360                 365

Gln Lys Val Glu Glu Met Val Gln Asn His Met Thr Tyr Ser Leu Gln
    370                 375                 380

Asp Val Gly Gly Asp Ala Asn Trp Gln Leu Val Val Glu Glu Gly Glu
385                 390                 395                 400

Met Lys Val Tyr Arg Arg Glu Val Glu Glu Asn Gly Ile Val Leu Asp
                405                 410                 415

Pro Leu Lys Ala Thr His Ala Val Lys Gly Val Thr Gly His Glu Val
            420                 425                 430

Cys Asn Tyr Phe Trp Asn Val Asp Val Arg Asn Asp Trp Glu Thr Thr
        435                 440                 445

Ile Glu Asn Phe His Val Val Glu Thr Leu Ala Asp Asn Ala Ile Ile

450                 455                 460

Ile Tyr Gln Thr His Lys Arg Val Trp Pro Ala Ser Gln Arg Asp Val
465                 470                 475                 480

Leu Tyr Leu Ser Val Ile Arg Lys Ile Pro Ala Leu Thr Glu Asn Asp
                485                 490                 495

Pro Glu Thr Trp Ile Val Cys Asn Phe Ser Val Asp His Asp Ser Ala
            500                 505                 510

Pro Leu Asn Asn Arg Cys Val Arg Ala Lys Ile Asn Val Ala Met Ile
        515                 520                 525

Cys Gln Thr Leu Val Ser Pro Pro Glu Gly Asn Gln Glu Ile Ser Arg
    530                 535                 540

Asp Asn Ile Leu Cys Lys Ile Thr Tyr Val Ala Asn Val Asn Pro Gly
545                 550                 555                 560

Gly Trp Ala Pro Ala Ser Val Leu Arg Ala Val Ala Lys Arg Glu Tyr
                565                 570                 575

Pro Lys Phe Leu Lys Arg Phe Thr Ser Tyr Val Gln Glu Lys Thr Ala
            580                 585                 590

Gly Lys Pro Ile Leu Phe
        595

<210>  2
<211>  624
<212>  PRT
<213>  HeLa cell

<400>  2

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Ala Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Ala Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Ser Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Asp Ser
            260                 265                 270

Trp Gln Lys Arg Leu Asp Lys Glu Thr Glu Lys Lys Arg Arg Thr Glu
        275                 280                 285

Glu Ala Tyr Lys Asn Ala Met Thr Glu Leu Lys Lys Lys Ser His Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Ser
            340                 345                 350

Leu Pro Ser Gly Asp Ala Phe Ser Ser Val Gly Thr His Arg Phe Val
        355                 360                 365

Gln Lys Pro Tyr Ser Arg Ser Ser Ser Met Ser Ser Ile Asp Leu Val
    370                 375                 380

Ser Ala Ser Asp Asp Val His Arg Phe Ser Ser Gln Val Glu Glu Met
385                 390                 395                 400

Val Gln Asn His Met Thr Tyr Ser Leu Gln Asp Val Gly Gly Asp Ala
                405                 410                 415

Asn Trp Gln Leu Val Val Glu Glu Gly Glu Met Lys Val Tyr Arg Arg
            420                 425                 430

Glu Val Glu Glu Asn Gly Ile Val Leu Asp Pro Leu Lys Ala Thr His
        435                 440                 445

Ala Val Lys Gly Val Thr Gly His Glu Val Cys Asn Tyr Phe Trp Asn
    450                 455                 460

Val Asp Val Arg Asn Asp Trp Glu Thr Thr Ile Glu Asn Phe His Val
465                 470                 475                 480

Val Glu Thr Leu Ala Asp Asn Ala Ile Ile Ile Tyr Gln Thr His Lys
                485                 490                 495

Arg Val Trp Pro Ala Ser Gln Arg Asp Val Leu Tyr Leu Ser Val Ile
            500                 505                 510

Arg Lys Ile Pro Ala Leu Thr Glu Asn Asp Pro Glu Thr Trp Ile Val
        515                 520                 525

Cys Asn Phe Ser Val Asp His Asp Ser Ala Pro Leu Asn Asn Arg Cys
    530                 535                 540

Val Arg Ala Lys Ile Asn Val Ala Met Ile Cys Gln Thr Leu Val Ser
545                 550                 555                 560

Pro Pro Glu Gly Asn Gln Glu Ile Ser Arg Asp Asn Ile Leu Cys Lys
                565                 570                 575

Ile Thr Tyr Val Ala Asn Val Asn Pro Gly Gly Trp Ala Pro Ala Ser
            580                 585                 590

Val Leu Arg Ala Val Ala Lys Arg Glu Tyr Pro Lys Phe Leu Lys Arg
        595                 600                 605

Phe Thr Ser Tyr Val Gln Glu Lys Thr Ala Gly Lys Pro Ile Leu Phe
    610                 615                 620

<210>  3
<211>  598

<212>  PRT
<213>  CHO-K1

<400>  3

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Thr Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Val Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Asn Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Glu Ser
            260                 265                 270

Trp Gln Lys Arg His Asp Lys Glu Met Glu Lys Arg Arg Arg Leu Glu
        275                 280                 285

Glu Ala Tyr Lys Asn Ala Met Ala Glu Leu Lys Lys Lys Pro Arg Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Pro
            340                 345                 350

Leu Pro Ser Gly Asp Ala Phe Ser Ser Val Gly Thr His Arg Phe Val
        355                 360                 365

Gln Lys Val Glu Glu Met Val Gln Asn His Met Thr Tyr Ser Leu Gln
    370                 375                 380

Asp Val Gly Gly Asp Ala Asn Trp Gln Leu Val Val Glu Glu Gly Glu
385                 390                 395                 400

Met Lys Val Tyr Arg Arg Glu Val Glu Glu Asn Gly Ile Val Leu Asp
                405                 410                 415

Pro Leu Lys Ala Thr His Ala Val Lys Gly Val Thr Gly His Glu Val
            420                 425                 430

Cys Asn Tyr Phe Trp Ser Val Asp Val Arg Asn Asp Trp Glu Thr Thr
        435                 440                 445

Ile Glu Asn Phe His Val Val Glu Thr Leu Ala Asp Asn Ala Ile Ile
    450                 455                 460

Ile Tyr Gln Thr His Lys Arg Val Trp Pro Ala Ser Gln Arg Asp Val
465                 470                 475                 480

Leu Tyr Leu Ser Ala Ile Arg Lys Ile Pro Ala Leu Thr Glu Asn Asp
                485                 490                 495

Pro Glu Thr Trp Ile Val Cys Asn Phe Ser Val Asp His Asp Ser Ala
            500                 505                 510

Pro Leu Asn Asn Arg Cys Val Arg Ala Lys Ile Asn Val Ala Met Ile
        515                 520                 525

Cys Gln Thr Leu Val Ser Pro Pro Glu Gly Asn Gln Glu Ile Ser Arg
    530                 535                 540

Asp Asn Ile Leu Cys Lys Ile Thr Tyr Val Ala Asn Val Asn Pro Gly
545                 550                 555                 560

Gly Trp Ala Pro Ala Ser Val Leu Arg Ala Val Ala Lys Arg Glu Tyr
                565                 570                 575

Pro Lys Phe Leu Lys Arg Phe Thr Ser Tyr Val Gln Glu Lys Thr Ala
            580                 585                 590

Gly Lys Pro Ile Leu Phe
        595

<210>  4
<211>  624
<212>  PRT
<213>  CHO-K1

<400>  4

Met Ser Asp Asn Gln Ser Trp Asn Ser Ser Gly Ser Glu Glu Asp Pro
1               5                   10                  15

Glu Thr Glu Ser Gly Pro Pro Val Glu Arg Cys Gly Val Leu Ser Lys
            20                  25                  30

Trp Thr Asn Tyr Ile His Gly Trp Gln Asp Arg Trp Val Val Leu Lys
        35                  40                  45

Asn Asn Thr Leu Ser Tyr Tyr Lys Ser Glu Asp Glu Thr Glu Tyr Gly
    50                  55                  60

Cys Arg Gly Ser Ile Cys Leu Ser Lys Ala Val Ile Thr Pro His Asp
65                  70                  75                  80

Phe Asp Glu Cys Arg Phe Asp Ile Ser Val Asn Asp Ser Val Trp Tyr
                85                  90                  95

Leu Arg Ala Gln Asp Pro Asp His Arg Gln Gln Trp Ile Asp Ala Ile
            100                 105                 110

Glu Gln His Lys Thr Glu Ser Gly Tyr Gly Ser Glu Ser Ser Leu Arg
        115                 120                 125

Arg His Gly Ser Met Val Ser Leu Val Ser Gly Ala Ser Gly Tyr Ser
    130                 135                 140

Ala Thr Ser Thr Ser Ser Phe Lys Lys Gly His Ser Leu Arg Glu Lys
145                 150                 155                 160

Leu Ala Glu Met Glu Thr Phe Arg Asp Ile Leu Cys Arg Gln Val Asp
                165                 170                 175

Thr Leu Gln Lys Tyr Phe Asp Val Cys Ala Asp Ala Val Ser Lys Asp
            180                 185                 190

Glu Leu Gln Arg Asp Lys Val Val Glu Asp Asp Glu Asp Asp Phe Pro
        195                 200                 205

Thr Thr Arg Ser Asp Gly Asp Phe Leu His Asn Thr Asn Gly Asn Lys
    210                 215                 220

Glu Lys Leu Phe Pro His Val Thr Pro Lys Gly Ile Asn Gly Ile Asp
225                 230                 235                 240

Phe Lys Gly Glu Ala Ile Thr Phe Lys Ala Thr Thr Ala Gly Ile Leu
                245                 250                 255

Ala Thr Leu Ser His Cys Ile Glu Leu Met Val Lys Arg Glu Glu Ser
            260                 265                 270

Trp Gln Lys Arg His Asp Lys Glu Met Glu Lys Arg Arg Arg Leu Glu
        275                 280                 285

Glu Ala Tyr Lys Asn Ala Met Ala Glu Leu Lys Lys Lys Pro Arg Phe
    290                 295                 300

Gly Gly Pro Asp Tyr Glu Glu Gly Pro Asn Ser Leu Ile Asn Glu Glu
305                 310                 315                 320

Glu Phe Phe Asp Ala Val Glu Ala Ala Leu Asp Arg Gln Asp Lys Ile
                325                 330                 335

Glu Glu Gln Ser Gln Ser Glu Lys Val Arg Leu His Trp Pro Thr Pro
            340                 345                 350

Leu Pro Ser Gly Asp Ala Phe Ser Ser Val Gly Thr His Arg Phe Val
        355                 360                 365

Gln Lys Pro Tyr Ser Arg Ser Ser Ser Met Ser Ser Ile Asp Leu Val
    370                 375                 380

Ser Ala Ser Asp Asp Val His Arg Phe Ser Ser Gln Val Glu Glu Met
385                 390                 395                 400

Val Gln Asn His Met Thr Tyr Ser Leu Gln Asp Val Gly Gly Asp Ala
                405                 410                 415

Asn Trp Gln Leu Val Val Glu Glu Gly Glu Met Lys Val Tyr Arg Arg
            420                 425                 430

Glu Val Glu Glu Asn Gly Ile Val Leu Asp Pro Leu Lys Ala Thr His
        435                 440                 445

Ala Val Lys Gly Val Thr Gly His Glu Val Cys Asn Tyr Phe Trp Ser
    450                 455                 460

Val Asp Val Arg Asn Asp Trp Glu Thr Thr Ile Glu Asn Phe His Val
465                 470                 475                 480

Val Glu Thr Leu Ala Asp Asn Ala Ile Ile Ile Tyr Gln Thr His Lys
                485                 490                 495

Arg Val Trp Pro Ala Ser Gln Arg Asp Val Leu Tyr Leu Ser Ala Ile
            500                 505                 510

Arg Lys Ile Pro Ala Leu Thr Glu Asn Asp Pro Glu Thr Trp Ile Val
        515                 520                 525

Cys Asn Phe Ser Val Asp His Asp Ser Ala Pro Leu Asn Asn Arg Cys
    530                 535                 540

Val Arg Ala Lys Ile Asn Val Ala Met Ile Cys Gln Thr Leu Val Ser
545                 550                 555                 560

Pro Pro Glu Gly Asn Gln Glu Ile Ser Arg Asp Asn Ile Leu Cys Lys
                565                 570                 575

Ile Thr Tyr Val Ala Asn Val Asn Pro Gly Gly Trp Ala Pro Ala Ser
            580                 585                 590

Val Leu Arg Ala Val Ala Lys Arg Glu Tyr Pro Lys Phe Leu Lys Arg
        595                 600                 605

Phe Thr Ser Tyr Val Gln Glu Lys Thr Ala Gly Lys Pro Ile Leu Phe
    610                 615                 620

<210>  5
<211>  1794
<212>  DNA
<213>  HeLa cell

<400>  5
atgtcggata atcagagctg gaactcgtcg ggctcggagg aggatccaga gacggagtct      60

gggccgcctg tggagcgctg cggggtcctc agtaagtgga caaactacat tcatgggtgg     120

caggatcgtt gggtagtttt gaaaaataat gctctgagtt actacaaatc tgaagatgaa     180

acagagtatg gctgcagagg atccatctgt cttagcaagg ctgtcatcac acctcacgat     240

tttgatgaat gtcgatttga tattagtgta aatgatagtg tttggtatct tcgtgctcag     300

gatccagatc atagacagca atggatagat gccattgaac agcacaagac tgaatctgga     360

tatggatctg aatccagctt gcgtcgacat ggctcaatgg tgtccctggt gtctggagca     420

agtggctact ctgcaacatc cacctcttca ttcaagaaag gccacagttt acgtgagaag 480
ttggctgaaa tggaaacatt tagagacatc ttatgtagac aagttgacac gctacagaag 540
tactttgatg cctgtgctga tgctgtctct aaggatgaac ttcaaaggga taaagtggta 600
gaagatgatg aagatgactt tcctacaacg cgttctgatg gtgacttctt gcatagtacc 660
aacggcaata aagaaaagtt atttccacat gtgacaccaa aaggaattaa tggtatagac 720
tttaaagggg aagcgataac ttttaaagca actactgctg gaatccttgc aacactttct 780
cattgtattg aactaatggt taaacgtgag gacagctggc agaagagact ggataaggaa 840
actgagaaga aaagaagaac agaggaagca tataaaaatg caatgacaga acttaagaaa 900
aaatcccact ttggaggacc agattatgaa gaaggcccta acagtctgat taatgaagaa 960
gagttctttg atgctgttga agctgctctt gacagacaag ataaaataga agaacagtca 1020
cagagtgaaa aggtgagatt acattggcct acatccttgc cctctggaga tgccttttct 1080
tctgtgggga cacatagatt tgtccaaaag gttgaagaga tggtgcagaa ccacatgact 1140
tactcattac aggatgtagg cggagatgcc aattggcagt tggttgtaga agaaggagaa 1200
atgaaggtat acagaagaga agtagaagaa aatgggattg ttctggatcc tttaaaagct 1260
acccatgcag ttaaaggcgt cacaggacat gaagtctgca attatttctg gaatgttgac 1320
gttcgcaatg actgggaaac aactatagaa aactttcatg tggtggaaac attagctgat 1380
aatgcaatca tcatttatca aacacacaag agggtgtggc ctgcttctca gcgagacgta 1440
ttatatcttt ctgtcattcg aaagatacca gccttgactg aaaatgaccc tgaaacttgg 1500
atagtttgta atttttctgt ggatcatgac agtgctcctc taaacaaccg atgtgtccgt 1560
gccaaaataa atgttgctat gatttgtcaa accttggtaa gcccaccaga gggaaaccag 1620
gaaattagca gggacaacat tctatgcaag attacatatg tagctaatgt gaaccctgga 1680
ggatgggcac cagcctcagt gttaagggca gtggcaaagc gagagtatcc taaatttcta 1740
aaacgtttta cttcttacgt ccaagaaaaa actgcaggaa agcctatttt gttc 1794

<210> 6
<211> 1872
<212> DNA
<213> HeLa cell

<400> 6
atgtcggata atcagagctg gaactcgtcg ggctcggagg aggatccaga gacggagtct 60
gggccgcctg tggagcgctg cggggtcctc agtaagtgga caaactacat tcatgggtgg 120
caggatcgtt gggtagtttt gaaaaataat gctctgagtt actacaaatc tgaagatgaa 180
acagagtatg gctgcagagg atccatctgt cttagcaagg ctgtcatcac acctcacgat 240
tttgatgaat gtcgatttga tattagtgta aatgatagtg tttggtatct tcgtgctcag 300
gatccagatc atagacagca atggatagat gccattgaac agcacaagac tgaatctgga 360
tatggatctg aatccagctt gcgtcgacat ggctcaatgg tgtccctggt gtctggagca 420
agtggctact ctgcaacatc cacctcttca ttcaagaaag gccacagttt acgtgagaag 480

ttggctgaaa tggaaacatt tagagacatc ttatgtagac aagttgacac gctacagaag 540
tactttgatg cctgtgctga tgctgtctct aaggatgaac ttcaaaggga taaagtggta 600
gaagatgatg aagatgactt tcctacaacg cgttctgatg gtgacttctt gcatagtacc 660
aacggcaata aagaaaagtt atttccacat gtgacaccaa aaggaattaa tggtatagac 720
tttaaagggg aagcgataac ttttaaagca actactgctg gaatccttgc aacactttct 780
cattgtattg aactaatggt taaacgtgag gacagctggc agaagagact ggataaggaa 840
actgagaaga aaagaagaac agaggaagca tataaaaatg caatgacaga acttaagaaa 900
aaatcccact ttggaggacc agattatgaa gaaggcccta acagtctgat taatgaagaa 960
gagttctttg atgctgttga agctgctctt gacagacaag ataaaataga agaacagtca 1020
cagagtgaaa aggtgagatt acattggcct acatccttgc cctctggaga tgccttttct 1080
tctgtgggga cacatagatt tgtccaaaag ccctatagtc gctcttcctc catgtcttcc 1140
attgatctag tcagtgcctc tgatgatgtt cacagattca gctcccaggt tgaagagatg 1200
gtgcagaacc acatgactta ctcattacag gatgtaggcg gagatgccaa ttggcagttg 1260
gttgtagaag aaggagaaat gaaggtatac agaagagaag tagaagaaaa tgggattgtt 1320
ctggatcctt taaaagctac ccatgcagtt aaaggcgtca caggacatga agtctgcaat 1380
tatttctgga atgttgacgt tcgcaatgac tgggaaacaa ctatagaaaa ctttcatgtg 1440
gtggaaacat tagctgataa tgcaatcatc atttatcaaa cacacaagag ggtgtggcct 1500
gcttctcagc gagacgtatt atatctttct gtcattcgaa agataccagc cttgactgaa 1560
aatgaccctg aaacttggat agtttgtaat ttttctgtgg atcatgacag tgctcctcta 1620
aacaaccgat gtgtccgtgc caaaataaat gttgctatga tttgtcaaac cttggtaagc 1680
ccaccagagg gaaaccagga aattagcagg gacaacattc tatgcaagat tacatatgta 1740
gctaatgtga accctggagg atgggcacca gcctcagtgt taagggcagt ggcaaagcga 1800
gagtatccta aatttctaaa acgttttact tcttacgtcc aagaaaaaac tgcaggaaag 1860
cctattttgt tc 1872

<210> 7
<211> 2473
<212> DNA
<213> CHO-K1

<400> 7
acgcggggag tgggcccggc aggaagatgg cggcggtagc ggaggtgtga gcggacctgg 60
gtctctgccg ctggcttggc tcttcctgtc ttcctcccct cctccctccc cgactgaggt 120
tggcacccag ggggccgagt tcaggtggcg gcgccgggcg cagcgcaggg gtcacggcca 180
cggccacggc ggctgacggc tggaagggca ggctttcttc gccgctcgtc ctccttccca 240
ggtccgctcg gtgtcaggcg cggcgacggc ggcgcagcgg gcgcgcttcc tccctcttcc 300
tgttccctca cgccccggag cgggcactct tggctgtgcc atcccccgac ccttcacccc 360

agggactggg cgcctgcact ggcgcagctc tcggagcggg ggccggtctc ctgctcgcct 420
gtcgcgcctc catgtcggat aaccagagct ggaactcgtc gggctcggag gaggatccgg 480
agacggagtc cgggccgcct gtggagcgct gcggggtcct cagcaagtgg acaaactata 540
ttcatgggtg gcaggatcgt tgggtagttt tgaaaaataa tactttgagt tactacaaat 600
ctgaagatga gacagagtac ggttgcaggg gatccatctg tcttagcaag gctgtgatca 660
cacctcatga ttttgatgaa tgtcggtttg atatcagtgt taatgatagt gtttggtatc 720
ttcgtgctca ggacccagat cacagacagc agtggataga tgccattgaa cagcacaaga 780
ctgaatcagg atatggatct gagtccagct tacgtagaca tggctcaatg gtgtcactgg 840
tgtctggagc aagtgggtac tctgctacat ccacatcttc attcaagaaa ggacacagtt 900
tacgtgagaa attggctgaa atggaaactt ttagagacat cttatgtaga caagttgaca 960
ctctccaaaa gtactttgat gtctgtgctg atgctgtctc caaggatgaa cttcaaaggg 1020
ataaagtggt agaagatgat gaagatgact tccctacaac tcgttctgat ggagactttt 1080
tgcacaatac caatggtaat aaggaaaaat tatttccaca tgtaaccccc aaaggaatta 1140
atggtataga ctttaaaggg gaagcaataa cttttaaagc aactactgct ggaatccttg 1200
ctacactttc tcattgtatt gaattaatgg taaaacggga agagagctgg caaaaaagac 1260
atgataagga aatggagaag agaagacgat tagaggaagc atacaagaat gcaatggcag 1320
agcttaagaa gaaaccccgt tttggagggc ctgattatga agaaggtccg aacagtctga 1380
ttaatgagga ggagttcttt gatgctgttg aagctgctct tgacagacaa gataaaatag 1440
aggaacagtc acagagcgag aaggtcaggt tacactggcc tacacctttg ccatctggag 1500
atgccttttc ttctgttggg acccatagat ttgtacaaaa ggttgaagag atggtacaga 1560
accacatgac ttactcatta caggatgtag gtggtgatgc gaattggcaa ctagttgtag 1620
aagaaggaga aatgaaggta tacagaagag aagtcgaaga aaatggaatt gttctggatc 1680
ctttgaaagc tacccatgca gttaaaggtg ttacaggaca cgaagtctgc aattactttt 1740
ggagtgttga tgttcgcaat gactgggaaa ctactataga aaacttccat gtagtggaaa 1800
cattagctga taatgcaatc atcatttatc aaacgcacaa gagagtgtgg cctgcttctc 1860
agagagatgt actgtatctt tctgctattc gaaagatccc agccttgact gagaacgacc 1920
ctgagacttg gatagtttgt aatttttctg tggatcatga cagcgctcct ctgaacaatc 1980
gatgtgtccg tgccaaaatc aatgttgcta tgatttgtca aaccttagta agcccaccag 2040
agggaaacca ggaaataagc agagacaaca ttctgtgcaa gattacatat gtagctaatg 2100
tgaacccagg aggatgggca ccagcctcgg tgttaagagc agtggcaaaa cgagaatatc 2160
ctaaatttct aaaacgtttt acttcttacg tccaagaaaa aactgcagga aaaccaattt 2220
tgttttagta tgtacagtga ctgaagcaag gctgtgtgac attccatgtt ggagaaagaa 2280
agaagaaaaa ttgagttctc taagctggaa cataggatct acagccttgt ccatggccca 2340
agaagaatca ttgcaatagt aaagctgggt atctaacact agccatctcc tgatagatct 2400

ccttgctcaa tgtgtaacta taaatacatg taaaatcaca tgcaaaaaaa aaaaaaaaaa 2460

aaaaaaaaaa aaa 2473

<210> 8
<211> 1872
<212> DNA
<213> CHO-K1

<400> 8
atgtcggata accagagctg gaactcgtcg ggctcggagg aggatccgga gacggagtcc 60

gggccgcctg tggagcgctg cggggtcctc agcaagtgga caaactatat tcatgggtgg 120

caggatcgtt gggtagtttt gaaaaataat actttgagtt actacaaatc tgaagatgag 180

acagagtacg gttgcagggg atccatctgt cttagcaagg ctgtgatcac acctcatgat 240

tttgatgaat gtcggtttga tatcagtgtt aatgatagtg tttggtatct tcgtgctcag 300

gacccagatc acagacagca gtggatagat gccattgaac agcacaagac tgaatcagga 360

tatggatctg agtccagctt acgtagacat ggctcaatgg tgtcactggt gtctggagca 420

agtgggtact ctgctacatc cacatcttca ttcaagaaag gacacagttt acgtgagaaa 480

ttggctgaaa tggaaacttt tagagacatc ttatgtagac aagttgacac tctccaaaag 540

tactttgatg tctgtgctga tgctgtctcc aaggatgaac ttcaaaggga taaagtggta 600

gaagatgatg aagatgactt ccctacaact cgttctgatg gagacttttt gcacaatacc 660

aatggtaata aggaaaaatt atttccacat gtaaccccca aaggaattaa tggtatagac 720

tttaaagggg aagcaataac ttttaaagca actactgctg gaatccttgc tacactttct 780

cattgtattg aattaatggt aaaacgggaa gagagctggc aaaaaagaca tgataaggaa 840

atggagaaga gaagacgatt agaggaagca tacaagaatg caatggcaga gcttaagaag 900

aaaccccgtt ttggagggcc tgattatgaa gaaggtccga acagtctgat taatgaggag 960

gagttctttg atgctgttga agctgctctt gacagacaag ataaaataga ggaacagtca 1020

cagagcgaga aggtcaggtt acactggcct acacctttgc catctggaga tgccttttct 1080

tctgttggga cccatagatt tgtacaaaag ccctatagtc gctcttcctc catgtcttcc 1140

attgatctag tcagtgcctc tgacgatgtt cacagattca gctcccaggt tgaagagatg 1200

gtacagaacc acatgactta ctcattacag gatgtaggtg gtgatgcgaa ttggcaacta 1260

gttgtagaag aaggagaaat gaaggtatac agaagagaag tcgaagaaaa tggaattgtt 1320

ctggatcctt tgaaagctac ccatgcagtt aaaggtgtta caggacacga agtctgcaat 1380

tacttttgga gtgttgatgt tcgcaatgac tgggaaacta ctatagaaaa cttccatgta 1440

gtggaaacat tagctgataa tgcaatcatc atttatcaaa cgcacaagag agtgtggcct 1500

gcttctcaga gagatgtact gtatctttct gctattcgaa agatcccagc cttgactgag 1560

aacgaccctg agacttggat agtttgtaat ttttctgtgg atcatgacag cgctcctctg 1620

aacaatcgat gtgtccgtgc caaaatcaat gttgctatga tttgtcaaac cttagtaagc 1680

ccaccagagg gaaaccagga aataagcaga gacaacattc tgtgcaagat tacatatgta 1740

gctaatgtga acccaggagg atgggcacca gcctcggtgt taagagcagt ggcaaaacga 1800

gaatatccta aatttctaaa acgttttact tcttacgtcc aagaaaaaac tgcaggaaaa 1860

ccaattttgt tt 1872

<210> 9
<211> 22
<212> DNA
<213> Artificial Sequence

<220>
<223> PCR primer

<400> 9
gccctcactc cttctctagg cg 22

<210> 10
<211> 27
<212> DNA
<213> Artificial Sequence

<220>
<223> PCR primer

<400> 10
cttaagctag cttgccaaac ctacagg 27

<210> 11
<211> 32
<212> DNA
<213> Artificial Sequence

<220>
<223> PCR primer

<400> 11
cagaattcac catgtcggat aatcagagct gg 32

<210> 12
<211> 56
<212> DNA
<213> Artificial Sequence

<220>
<223> PCR primer

<400> 12
cagaattcac catggactac aaggacgacg acaaaatgtc ggataatcag agctgg 56

<210> 13
<211> 32
<212> DNA
<213> Artificial Sequence

<220>
<223> PCR primer

<400> 13
ggctcgagct agaacaaaat aggctttcct gc 32

<210> 14
<211> 53
<212> DNA
<213> Artificial Sequence

<220>
<223> PCR primer

<400> 14
ggctcgagct atttgtcgtc gtccttgtag tcgaacaaaa taggctttcc tgc 53

<210> 15
<211> 75
<212> DNA
<213> Artificial Sequence

<220>
<223> PCR primer

<400> 15
cagaggcact gactagatca atggaagaca tggaggaaga gcgactatag ggcttttgga 60

caaatctatg tgtcc 75

<210> 16
<211> 72
<212> DNA
<213> Artificial Sequence

<220>
<223> PCR primer

<400> 16
ccattgatct agtcagtgcc tctgatgatg ttcacagatt cagctcccag gttgaagaga 60

tggtgcagaa cc 72

<210> 17
<211> 37
<212> DNA
<213> Artificial Sequence

<220>
<223> PCR primer

<400> 17
cagaattcac catggaatct ggatatggat ctgaatc 37

<210> 18
<211> 32
<212> DNA
<213> Artificial Sequence

<220>
<223> PCR primer

<400> 18
ggctcgagct attggacaaa tctatgtgtc cc 32

<210> 19
<211> 33
<212> DNA
<213> Artificial Sequence

<220>
<223>  PCR primer

<400>  19
ctcttcaacc ttagtcttgt gctgttcaat ggc                                33

<210>  20
<211>  33
<212>  DNA
<213>  Artificial Sequence

<220>
<223>  PCR primer

<400>  20
cagcacaaga ctaaggttga agagatggtg cag                                33

<210>  21
<211>  29
<212>  DNA
<213>  Artificial Sequence

<220>
<223>  PCR primer

<400>  21
cacaagcttc ggataatcag agctggaac                                     29

<210>  22
<211>  38
<212>  DNA
<213>  Artificial Sequence

<220>
<223>  PCR primer

<400>  22
ggctcgagct aagatctagt cttgtgctgt tcaatggc                           38

<210>  23
<211>  35
<212>  DNA
<213>  Artificial Sequence

<220>
<223>  PCR primer

<400>  23
cacaagcttc ggaatctgga tatggatctg aatcc                              35

<210>  24
<211>  38
<212>  DNA
<213>  Artificial Sequence

<220>
<223>  PCR primer

<400>  24
ggctcgagct aagatctttg gacaaatcta tgtgtccc                           38

<210>  25
<211>  32
<212>  DNA
<213>  Artificial Sequence

<220>
<223>  PCR primer

<400>  25
cacaagcttc gaaggttgaa gagatggtgc ag                                    32


<210>  26
<211>  38
<212>  DNA
<213>  Artificial Sequence

<220>
<223>  PCR primer

<400>  26
ggctcgagct aagatctgaa caaaataggc tttcctgc                              38


<210>  27
<211>  34
<212>  DNA
<213>  Artificial Sequence

<220>
<223>  PCR primer

<400>  27
gagaaagtgt agcaaggatt ccagcagtag ttgc                                  34


<210>  28
<211>  32
<212>  DNA
<213>  Artificial Sequence

<220>
<223>  PCR primer

<400>  28
gaagatgact tccctacaac tcgttctgat gg                                    32


<210>  29
<211>  24
<212>  DNA
<213>  Artificial Sequence

<220>
<223>  PCR primer

<400>  29
tagaattcac tggcgcagct ctcg                                             24


<210>  30
<211>  27
<212>  DNA
<213>  Artificial Sequence

<220>
<223>  PCR primer

<400>  30
ggctcgagga atgtcacaca gccttgc                                          27

<210>  31
<211>  19
<212>  DNA
<213>  Artificial Sequence

<220>
<223>  PCR primer

<400>  31
aagatcccag ccttgactg                                                   19

<210>  32
<211>  29
<212>  DNA
<213>  Artificial Sequence

<220>
<223>  PCR primer

<400>  32
ggctcgagga acaaaatagg ctttcctgc                                        29